# EUROPEAN PATENT APPLICATION

(11) **EP 4 442 275 A1**
(43) Date of publication of application: **09.10.2024**
(21) Application number: 23167155.3
(22) Date of filing: 06.04.2023
(51) Int. Cl.: A61K 39/395, A61K 49/00, A61P 19/02, C07K 16/18

(54) **ANTI-TYPE II COLLAGEN ANTIBODIES**

(71) Applicant: ModiQuest B.V., 5349 AB Oss (NL)
(72) Inventor: The designation of the inventor has not yet been filed
(74) Representative: J A Kemp LLP

(57) **Abstract**

The invention provides anti-collagen type II antibodies. The antibodies are able to distinguish between mild and severe disease-induced human cartilage damage. The invention further provides methods for using the antibodies for monitoring disease progression and delivering targeted therapeutics.

## Description

### Field of invention

The invention relates to antibodies that are useful for detecting damaged cartilage, for example in osteoarthritis, and for methods of treatment, screening potential therapeutics, diagnosis, prognosis and monitoring disease progression.

### Background of the invention

Osteoarthritis (OA) and_Rheumatoid arthritis (RA) are chronic inflammatory and autoimmune diseases characterized by synovial inflammation and destruction of cartilage. The cartilage matrix consists of a network of collagen type II in which proteoglycans are embedded. During early disease, proteoglycans are degraded by metalloproteinases, exposing the collagen fibers. This early cartilage damage is reversible. Initiating effective therapy within the first 2 years of clinical symptoms leads to a better long-term disease outcome. However, OA and RA are heterogeneous diseases with large variations in onset, rate of progression and response to treatment. Hence, early detection and personalized treatment regimes are desirable.

Imaging modalities such as radiography, computed tomography (CT), ultrasonography and magnetic resonance imaging (MRI) can visualize cartilage destruction but are not very sensitive and mainly allow the identification of advanced cartilage damage (Lim et al. (2020) Osteroarthritis and Cartilage 28: 874-884).

### Summary of the invention

The inventors have identified antibodies that are able to distinguish between damaged and un-damaged bovine and human cartilage. Specific antibodies retain the ability to distinguish between damaged and un-damaged bovine and human cartilage when the antibodies are fully humanized. The antibodies can distinguish between damaged and undamaged human collagen explants in mice. Moreover, the antibodies can be used to distinguish between mildly and severely damaged *ex vivo* human cartilage from patients. Hence, disease-induced damage can also be detected, imaged and quantified. Such antibodies may be used to detect early cartilage damage, facilitate early diagnosis, monitor disease progression or response to treatment, for non-invasive testing of possible new therapeutics (such as multispecific antibodies), or delivery of therapeutics to the site of damage, allowing for systemic treatment (instead of intra-articular), more spatially targeted treatment, and lower side-effects.

Accordingly, an aspect of the invention provides an antibody or an antigen-binding fragment thereof that binds to collagen type II, wherein the antibody comprises a set of three heavy chain complementarity determining regions (CDRH1, CDRH2 and CDRH3) and three light chain complementarity determining regions (CDRL1, CDRL2 and CDRL3), wherein the set of CDRH1, CDRH2, CDRH3 amino acid sequences is selected from SEQ ID NOs: 35, 37 and 39; SEQ ID NOs: 3, 5 and 7; SEQ ID NOs: 19, 21 and 23; SEQ ID NOs: 27, 29 and 31; SEQ ID NOs: 43, 45 and 47; SEQ ID NOs: 67, 69 and 71; SEQ ID NOs: 83, 85 and 87; and SEQ ID NOs: 91, 93 and 95; and the set of CDRL1, CDRL2, CDRL3 amino acid sequences is selected from: SEQ ID NOs: 51, 53 and 55; SEQ ID NOs: 11, 13 and 17; SEQ ID NOs: 59, 61 and 63; SEQ ID NOs: 75, 77 and 79; SEQ ID NOs: 99, 101 and 103; SEQ ID NOs: 107, 109 and 111; and SEQ ID NOs: 115, 117 and 119. The antibody may comprise a set of CDRH1, CDRH2, CDRH3, CDRL1, CDRL2, CDRL3 amino acid sequences is selected from: SEQ ID NOs: 35, 37, 39 51, 53 and 55; SEQ ID NOs: 3, 5, 7, 11, 13 and 15; SEQ ID NOs: 19, 21, 23; SEQ ID NOs: 27, 29, 31; SEQ ID NOs: 43, 45, 47; SEQ ID NOs: 67, 69, 71; SEQ ID NOs: 83, 85, 87; and SEQ ID NOs: 91, 93, 95. The antibody or fragment thereof may comprise a heavy chain variable region (HCVR) having at least 80% amino acid sequence identity to any one of SEQ ID NOs: 33, 17, 25, 41, 65, 81 and 89, or comprising the amino acid sequence of any one of SEQ ID NOs: 33, 17, 25, 41, 65, 81 and 89; a light chain variable region (LCVR) having at least 80% amino acid sequence identity to any one of SEQ ID NOs: 49, 9, 57, 73, 97, 105 and 113, or comprising the amino acid sequence of any one of SEQ ID NOs: 49, 9, 57, 73, 97, 105 and 113; and/or a HCVR and LCVR pair having a sequence pair selected from SEQ ID NOs: 33 and 49, SEQ ID NOs: 17 and 9, SEQ ID NOs: 25 and 57, SEQ ID NOs: 41 and 73, SEQ ID NOs: 65 and 97, SEQ ID NOs: 81 and 105, and SEQ ID NOs: 89 and 113.

In another aspect, the invention provides an antibody or an antigen-binding fragment thereof that binds to collagen type II, wherein the antibody comprises a set of three heavy chain complementarity determining regions (CDRH1, CDRH2 and CDRH3) and/or three light chain complementarity determining regions (CDRL1, CDRL2 and CDRL3), of any of the antibodies shown in Table 2 and/or Table 3 herein.

The antibody or fragment thereof is useful for detecting cartilage damage or loss of cartilage proteoglycan and for detecting or monitoring disease, particularly OA or RA. Hence, the coupled to a detectable label, such as a fluorescent moiety, a magnetic spin label, such as a superparamagnetic iron oxide nanoparticle (SPION), or a radio-isotope label, such as a radionucleotide.

The antibody may be multispecific or bispecific antibody. Hence, the antibody may bind to a second antigen that is useful for targeting the antibody to sites of interest or for therapy. In some embodiments, the antibody comprises a second antigen binding domain that binds to a growth factor.

The antibody may be linked, fused, conjugated or used to deliver a therapeutic agent. In one embodiment, the antibody or fragment thereof is fused to an anti-inflammatory cytokine. In another embodiment, the antibody is conjugated to an immunoliposome containing a therapeutic agent for treating osteoarthritis or rheumatoid arthritis.

The invention also provides a pharmaceutical composition comprising an antibody or fragment according to the invention. The composition typically comprises at least one pharmaceutically acceptable diluent or carrier.

In a further aspect, the invention provides the antibody or fragment or the pharmaceutical composition for use in a method for treatment of a human or animal body by therapy. The invention also provides the antibody or fragment the pharmaceutical composition according to the invention, for use in a method for treating osteoarthritis or rheumatoid arthritis.

In a further aspect, the invention provides a method of treating a subject (in need thereof), the method comprising administering (a therapeutically effective amount of) the antibody or fragment or the pharmaceutical composition to the subject. The invention also provides a method of treating osteoarthritis or rheumatoid arthritis in a subject (in need thereof), the method comprising administering (a therapeutically effective amount of) the antibody or fragment or the pharmaceutical composition to the subject.

In a further aspect, the invention provides the use of the antibody or fragment in the manufacture of a medicament for treating a subject, or for treating osteoarthritis or rheumatoid arthritis.

In a further aspect, the invention provides a method of detecting, quantifying or imaging cartilage damage or loss of cartilage proteoglycan, the method comprising: (i) contacting the cartilage with the antibody or fragment thereof; and (ii) detecting, quantifying or imaging the presence of the antibody bound to the cartilage, wherein the presence of the antibody-antigen complex, or increased presence of the antibody-antigen complex compared to a reference, indicates cartilage damage or loss of cartilage proteoglycan.

In a further aspect, the invention provides a method of diagnosing, prognosing or monitoring progression or regression of osteoarthritis or rheumatoid arthritis in a subject, the method comprising (i) administering to the subject an antibody or fragment thereof coupled to an detectable label according to claim 2 or claim 3; and (ii) detecting, quantifying or imaging the antibody or fragment thereof coupled to detectable label bound to cartilage in the subject, wherein the presence of the antibody bound to cartilage, or of increased antibody bound to cartilage compared to a reference, indicates osteoarthritis or rheumatoid arthritis in the subject;

In a further aspect, the invention provides a method of diagnosing, prognosing or monitoring progression or regression of osteoarthritis or rheumatoid arthritis in a subject, the method comprising (i) administering to the subject an antibody or fragment thereof coupled to an detectable label according to claim 2 or claim 3; and (ii) detecting, quantifying or imaging the antibody or fragment thereof coupled to detectable label bound to cartilage in the subject, wherein increased antibody bound to cartilage in the subject compared to a reference or to earlier measurements from the same subject indicates progression of osteoarthritis or rheumatoid arthritis or a negative prognosis.

In a further aspect, the invention provides a method of diagnosing, prognosing or monitoring progression or regression of osteoarthritis or rheumatoid arthritis in a subject, the method comprising (i) administering to the subject an antibody or fragment thereof coupled to an detectable label according to claim 2 or claim 3; and (ii) detecting, quantifying or imaging the antibody or fragment thereof coupled to detectable label bound to cartilage in the subject, wherein decreased antibody bound to cartilage in the subject compared to a reference or to earlier measurements from the same subject indicates regression of osteoarthritis or rheumatoid arthritis or a positive prognosis in the subject.

In a further aspect, the invention provides a method of monitoring the response of a subject to a treatment for osteoarthritis or rheumatoid arthritis, wherein the subject has been administered an antibody according to claim 2 or claim 3, the method comprising detecting, quantifying or imaging the antibody bound to the cartilage in the subject (a) before administration of the treatment and (b) after administration of the treatment and comparing (a) and (b).

In a further aspect, the invention provides the antibody or fragment thereof for use in any of the methods above.

In a further aspect, the invention provides one or more polynucleotides encoding the antibody or fragment thereof.

In a further aspect, the invention provides one or more vectors comprising the polynucleotide or polynucleotides encoding the antibody of fragment thereof.

In a further aspect, the invention provides a host cell comprising said vector or vectors.

In a further aspect, the invention provides a method for producing the antibody or fragment, the method comprising culturing said host cell and isolating the antibody or fragment thereof from the culture.

The disclosure will now be described in more detail, by way of example and not limitation, and by reference to the accompanying drawings. Many equivalent modifications and variations will be apparent, to those skilled in the art when given this disclosure. Accordingly, the exemplary embodiments of the disclosure set forth are considered to be illustrative and not limiting. Various changes to the described embodiments may be made without departing from the scope of the invention, which is defined by the claims. All documents cited herein, whether supra or infra, are expressly incorporated by reference in their entirety.

### Brief description of the figures

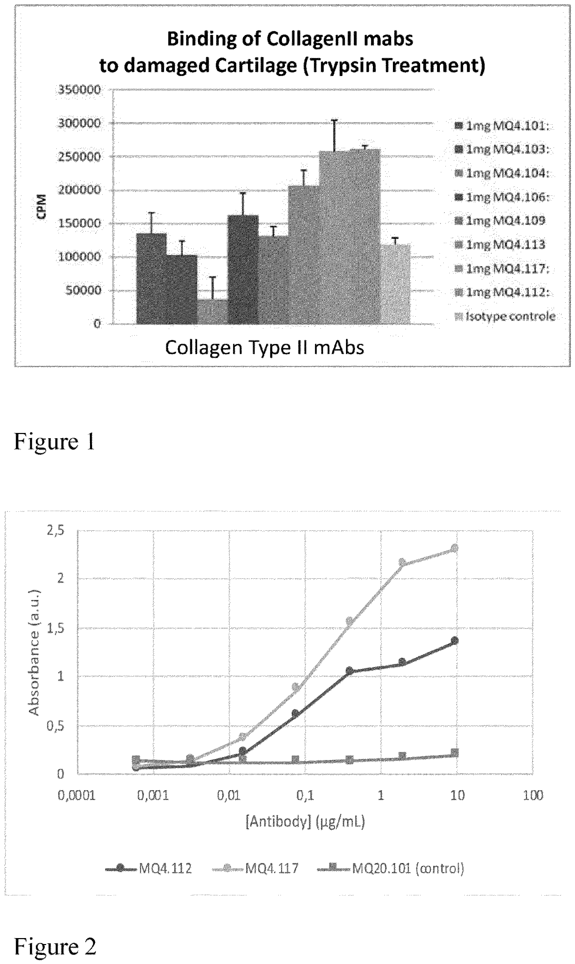

**Fig 1** **-** Binding of 8 different 125l labeled collagen type II antibodies and an isotype control antibody to bovine cartilage explants. Well-defined bovine cartilage explants were damaged by 2.5% trypsin treatment and additionally incubated with 25l labeled antibody (1 mg/ml). Three out of 8 collagen type II antibodies showed higher binding to damaged cartilage when compared to the isotype control. n=6 per group, ** p<0.01, *** p<0.0001 when using a one-way ANOVA.
**Fig 2** - Binding of Hybridoma produced antibodies to Bovine Collagen type II (10 µg/mL). Wells incubated with MQ4.112/MQ4.117 were developed for 3:15 minutes, wells with MQ20.101 were developed for 20 minutes

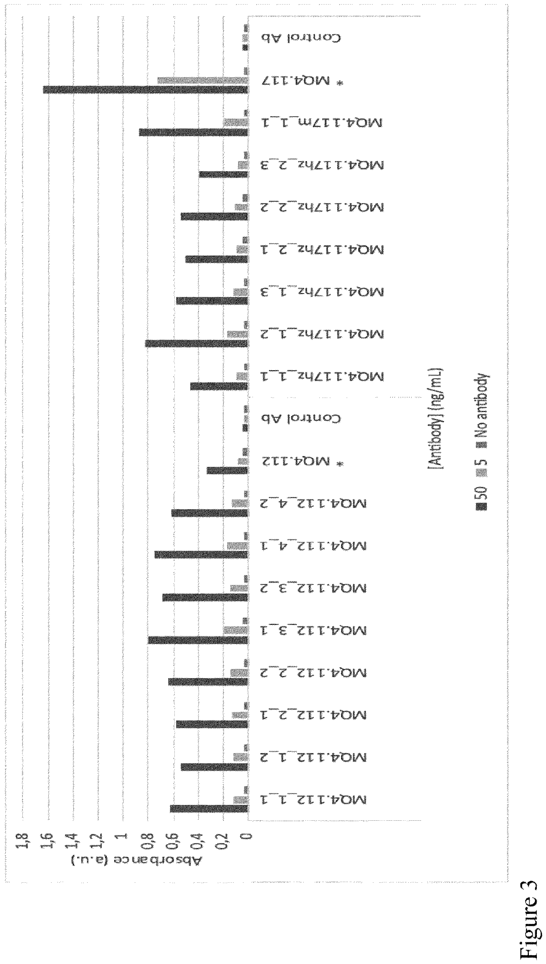

**Fig 3** - Binding of mouse, chimera and humanized antibodies to Bovine Collagen type II (10 µg/mL). * MQ4.112 and MQ4.117 are incubated with an anti-mouse IgG-HRP antibody, others with anti-human IgG-HRP.

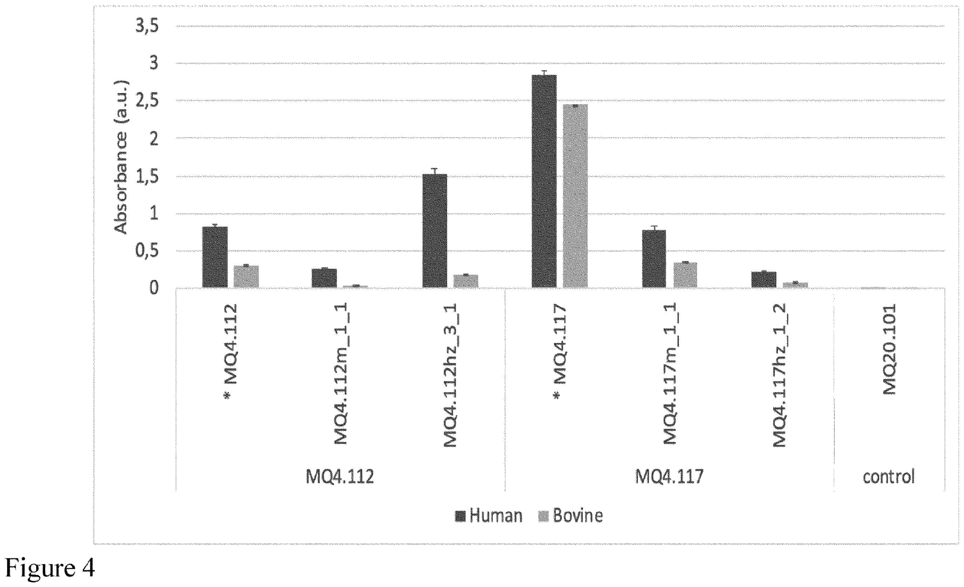

**Fig 4** - Binding of mouse, chimera and humanized antibodies (0.5 µg/mL) to Bovine and Human Collagen type II (20 µg/mL). * MQ4.112 and MQ4.117 are incubated with an anti-mouse IgG-HRP antibody, others with anti-human IgG-HRP.

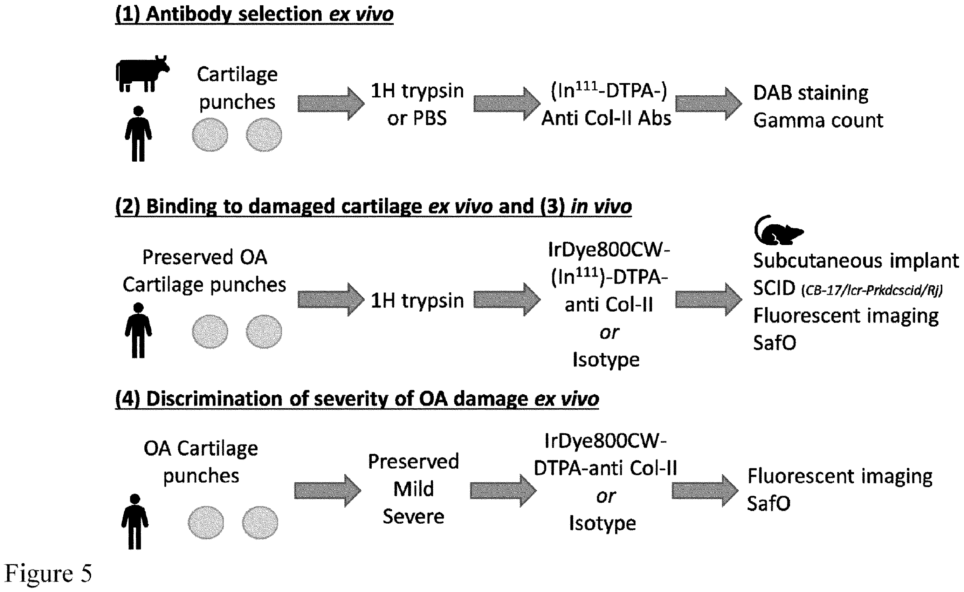

**Fig 5** - Schematic summarising experimental validation.

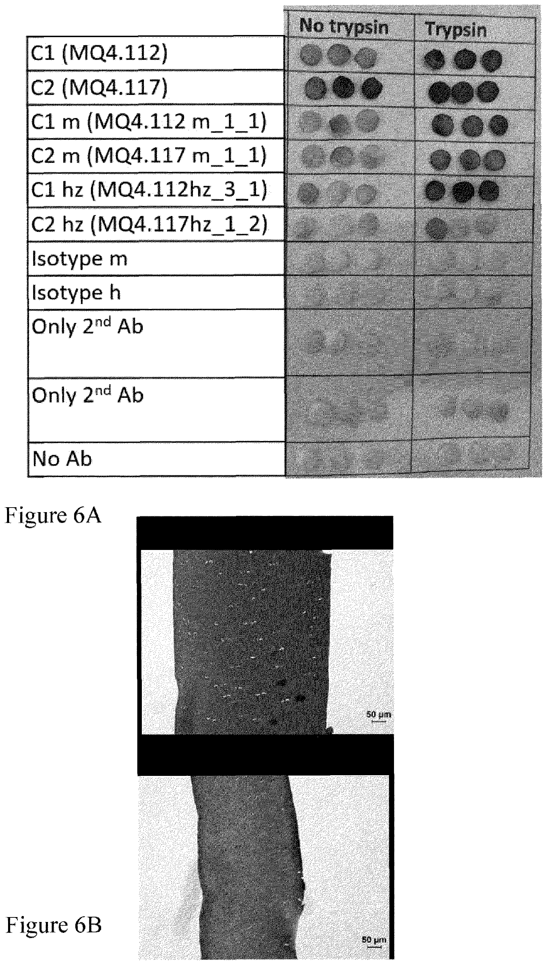

**Fig 6** **- Clone selection of bovine cartilage punches**. A) Bovine cartilage (untreated or treated with trypsin) was exposed to the different antibodies, followed by exposure to HRP-labeled secondary antibodies and 3,3'-diaminobenzidine (DAB) was used to stain cartilage punches. Picture shows increased binding of COLL II antibodies to bovine cartilage after trypsin treatment, clone MQ4.112 seems more specific to detect trypsin induced cartilage damage. B) Example pictures (10x) of a SafO staining from cartilage sections of condition 5 (MQ4.112hz_3_1) treated with or without Trypsin reveals trypsin-induced proteoglycan depletion. C= clone, m= murine, vc= variable domain of original mouse clone in human backbone and hz= fully humanized.

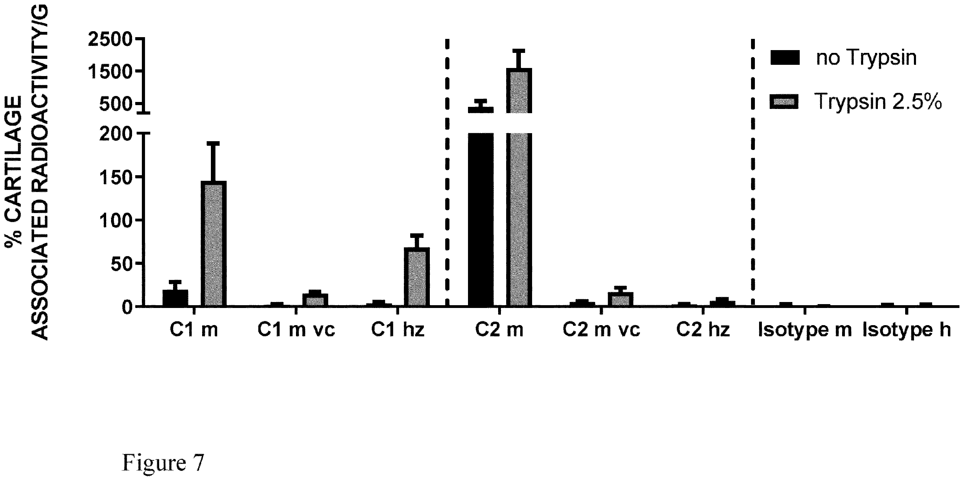

**Fig 7** - **Weight corrected cartilage associated radioactivity.** The cartilage associated radioactivity measured by gamma-counting is corrected for the weight of individual cartilage explants. MQ4.112 shows higher binding to trypsin-treated cartilage (grey bars) compared to untreated punches (black bars). Bar graphs show MEAN±SD (n=3 explants).
**Fig 8** - **Binding of [¹¹¹In]In-DTPA-MQ4.112hz_3_1 or [¹¹¹In]In-DTPA-2.201a (isotype) to trypsin-damaged human cartilage.** The cartilage associated radioactivity/gram of [¹¹¹In]In-DTPA-MQ4.112hz_3_1 is higher in trypsin-damaged human cartilage compared to non-treated explants and compared to isotype. The scatter plot depicts MEAN±SD, a One-way ANOVA Bonferroni's Multiple Comparison Test was used to test for statistical significance, ^{∗∗∗}*p*<0.001.

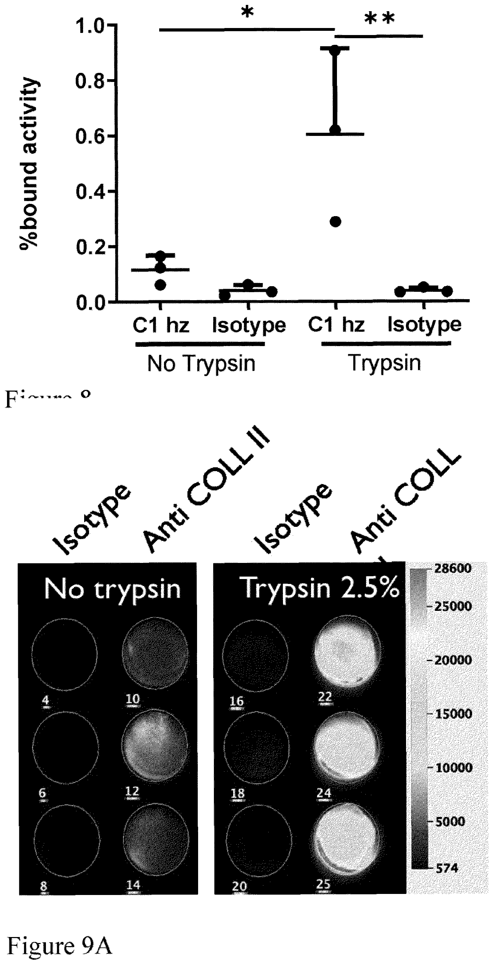

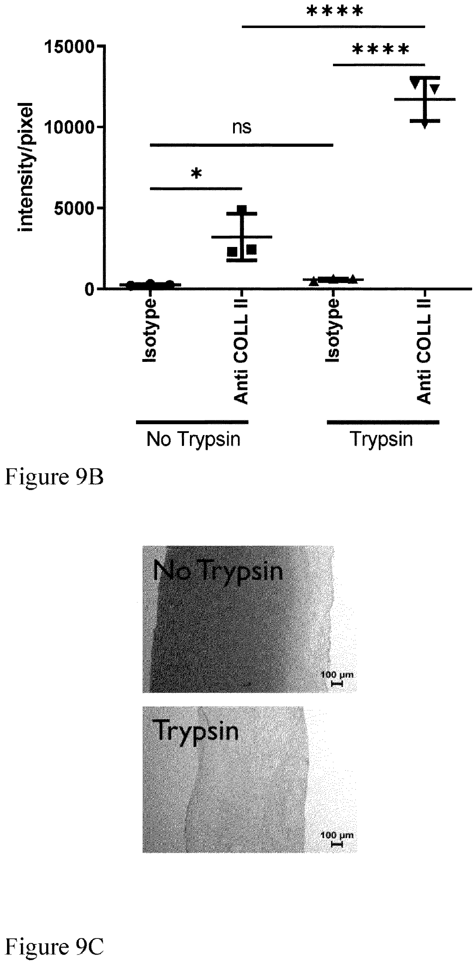

**Fig 9** - **DTPA-IrDye 800CW anti-COLL II (MQ4.112 hz3_1) binds to damaged cartilage.** Human cartilage of the preserved region of an OA patient that underwent joint replacement surgery was treated with or without trypsin and exposed to the DTPA-IrDye 800CW anti-COLL II antibody (MQ4.112 hz3_1) or the DTPA-IrDye 800CW isotype (2.201a) control. A) Picture of the fluorescent signal in these explants. B) Quantification of the fluorescent intensity/pixel. Trypsin treatment increases the fluorescent signal of the anti-COLL II antibody bound to human cartilage, while the isotype signal was very low in both trypsin-treated and untreated human cartilage explants. C) Representative picture of a SafO staining in these samples which verifies trypsin-induced proteoglycan depletion. The scatter plot depicts MEAN±SD, a One-way ANOVA Bonferroni's Multiple Comparison Test was used to test for statistical significance, ^{∗}*p*<0.05, ^{∗∗∗∗}*p*<0.0001.

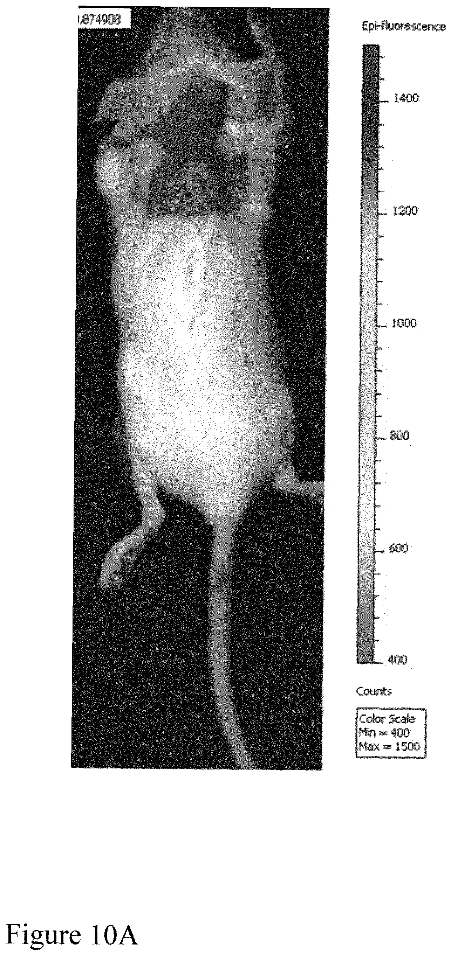

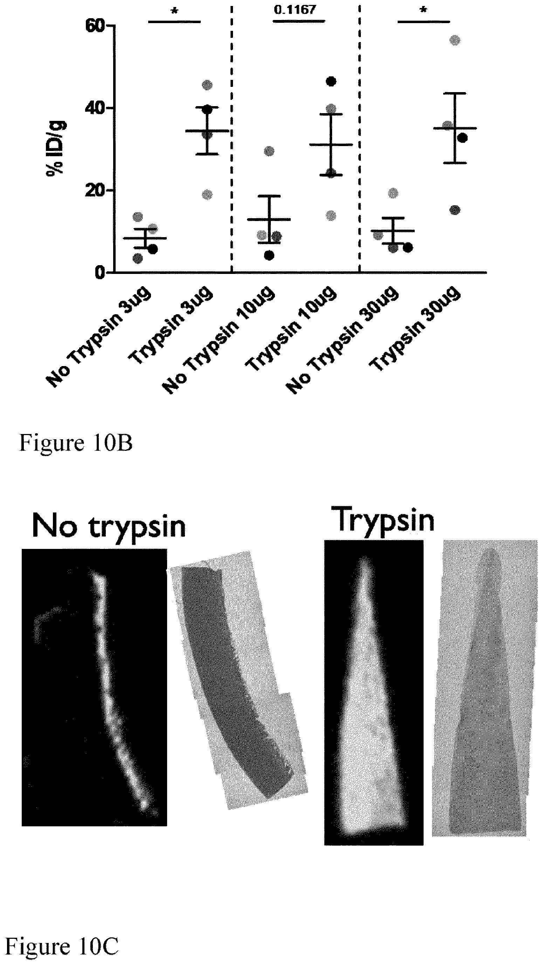

**Fig 10** - **[¹¹¹In]In-DTPA-IrDye 800CW anti-COLL II (MQ4.112hz3_1) can be used to determine cartilage damage *in vivo.*** A) IVIS imaging shows higher binding of the anti-COLL II antibody to trypsin-induced damaged cartilage (implanted on the right shoulder) compared to the explants left untreaded (implanted on the left shoulder). B) The signal of the anti-COLL II antibody per gram of implanted explant at 48h was higher in trypsin treated cartilage compared to untreated explants for all antibody dosages (3, 10 or 30 µg/mouse) (n=4 untreated and n=4 trypsin-treated cartilage explants/group). C) Example pictures of the fluorescent signal in sections of cartilage explants (Odyssey imaging) implanted in SCID mice which were untreated (left) or treated with trypsin (right) and SafO staining of the same section. This reveals more binding to trypsin treated explants and shows binding to the topical part of the untreated cartilage explant (derived from macroscopically preserved OA cartilage). The scatter plot depicts MEAN±SD (each color represents a different cartilage donor), a paired Student's T-test was used to test for statistical significance, ^{∗}*p*<0.05.

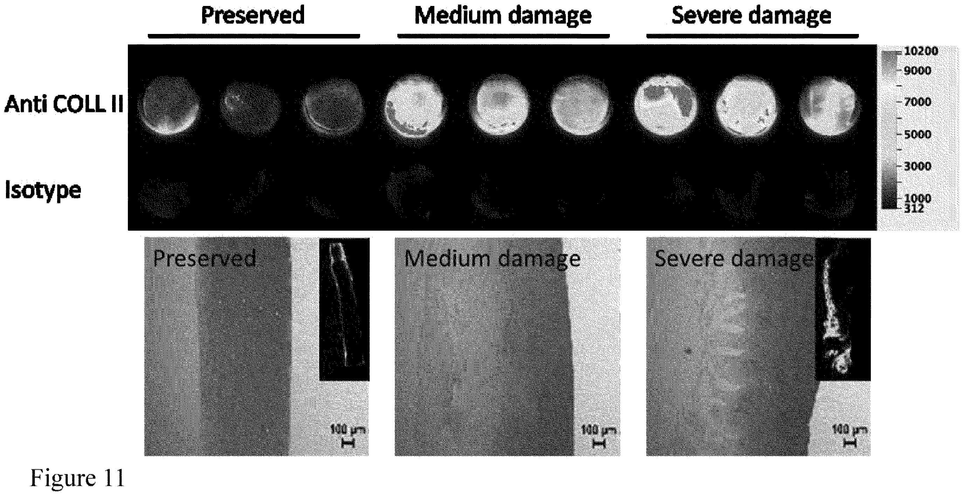

**Fig 11** - **The DTPA-IrDye 800CW anti-COLL II antibody can be used to detect severity of OA-damage *in vitro.*** Fluorescent signal (Odyssey imaging) from the anti-COLL II antibody increases when the macroscopically cartilage damage increases. Increased damage in these explants was verified with SafO staining. The localization of the fluorescent signal revealed that antibody binding localizes where SafO staining is lost, which indicates proteoglycan depletion.

### Description of the Sequences

SEQ ID NOs: 1 to 120 are included in the sequence listing as filed and are described in Table 2.

### Detailed description of the invention

### Antibodies of the invention

An antibody of the invention specifically binds to collagen type II.

In one embodiment, an antibody of the invention may comprise the six CDRs, as listed in Table 2, of any of the antibodies listed in Table 3, or any combination thereof.

The antibody may comprise the heavy chain variable region (HCVR) and/or the light chain variable region (LCVR), as listed in Table 2, of any of the antibodies listed in Table 3, or any combination thereof.

The antibody may comprise the three heavy chain CDR sequences, or the HCVR sequence, of any of the 4.112hz heavy chains listed in Table 2, combined with the three light chain CDR sequences, or the LCVR sequence, of any of the 4.112hz heavy chains listed in Table 2.

The antibody may comprise the three heavy chain CDR sequences, or the HCVR sequence, of any of the 4.117hz heavy chains listed in Table 2, combined with the three light chain CDR sequences, or the LCVR sequence, of any of the 4.117hz heavy chains listed in Table 2.

Hence, in some embodiments the antibody may comprise a set of CDRH1, CDRH2, CDRH3, CDRL1, CDRL2, CDRL3 amino acid sequences selected from (a) SEQ ID NOs: 35, 37, 39 51, 53 and 55 (MQ4.112hz_3_1); (b) SEQ ID NOs: 3, 5, 7, 11, 13 and 15 (MC14.112); (c) SEQ ID NOs: 83, 85, 87, 107, 109 and 111 (MQ4.117hz_1_2); and (d) SEQ ID NOs: 67, 69, 71, 75, 77 and 79 (MQ4.117). In some embodiments the antibody may comprise the HCVR and LCVR amino acid sequences of (a) SEQ ID NOs: 33 and 49; (MQ4.112hz_3_1); (b) SEQ ID NOs: 1 and 9 (MC14.112); (c) SEQ ID NOs: 81 and 105 (MQ4.117hz_1_2); and (d) SEQ ID NOs: 65 and 74 (MC14.117).

In some embodiments the antibody may comprise a set of CDRH1, CDRH2, CDRH3, CDRL1, CDRL2, CDRL3 amino acid sequences selected from (a) SEQ ID NOs: 35, 37, 39, 51, 53 and 55 (MQ4.112hz_3_1); (b) 35, 37, 39, 59, 61 and 63 (MQ4.112hz_3_2); (c) SEQ ID NOs: 19, 21, 23, 51, 53 and 55 (MQ4.112hz_1_1); (d) SEQ ID NOs: 19, 21, 23, 59, 61 and 63 (MQ4.112hz_1_2); (e) SEQ ID NOs: 27, 29, 31, 51, 53 and 55 (MQ4.112hz 2_1); (f) SEQ ID NOs: 27, 29, 31, 59, 61 and 63 (MQ4.112hz_2_2); (g) SEQ ID NOs: 43, 45, 47, 51, 53 and 55 (MQ4.112hz_4_1); (h) SEQ ID NOs: 43, 45, 47, 59, 61 and 63 (MQ4.112hz_4_2). In some embodiments the antibody may comprise the HCVR and LCVR amino acid sequences of (a) SEQ ID NOs: 33 and 49; (MQ4.112hz_3_1); (b) 33 and 57 (MQ4.112hz_3_2); (c) SEQ ID NOs: 17 and 49 (MQ4.112hz_1_1); (d) SEQ ID NOs: 17 and 57 (MQ4.112hz_1_2); (e) SEQ ID NOs: 25 and 49 (MQ4.112hz_2_1); (f) SEQ ID NOs: 25 and 49 (MQ4.112hz_2_2); (g) SEQ ID NOs: 41 and 49 (MQ4.112hz_4_1); and (h) SEQ ID NOs: 41 and 57 (MQ4.112hz_4_2).

In some embodiments the antibody may comprise a set of CDRH1, CDRH2, CDRH3, CDRL1, CDRL2, CDRL3 amino acid sequences selected from (a) SEQ ID NOs: 35, 37, 39, 51, 53 and 55 (MQ4.112hz_3_1); (b) 35, 37, 39, 59, 61 and 63 (MQ4.112hz_3_2); (c) SEQ ID NOs: 19, 21, 23, 51, 53 and 55 (MQ4.112hz_1_1); (d) SEQ ID NOs: 27, 29, 31, 51, 53 and 55 (MQ4.112hz_2_1); and (e) SEQ ID NOs: 43, 45, 47, 51, 53 and 55 (MQ4.112hz_4_1). In some embodiments the antibody may comprise the HCVR and LCVR amino acid sequences of (a) SEQ ID NOs: 33 and 49; (MQ4.112hz_3_1); (b) 33 and 57 (MQ4.112hz_3_2); (c) SEQ ID NOs: 17 and 49 (MQ4.112hz_1_1); (d) SEQ ID NOs: 25 and 49 (MQ4.112hz_2_1); and (e) SEQ ID NOs: 41 and 49 (MQ4.112hz_4_1).

In some embodiments the antibody may comprise a set of CDRH1, CDRH2, CDRH3, CDRL1, CDRL2, CDRL3 amino acid sequences selected from (a) SEQ ID NOs: 35, 37, 39, 51, 53 and 55 (MQ4.112hz_3_1) and (b) 35, 37, 39, 59, 61 and 63 (MQ4.112hz_3_2). In some embodiments the antibody may comprise the HCVR and LCVR amino acid sequences of (a) SEQ ID NOs: 33 and 49; (MQ4.112hz_3_1); (b) 33 and 57 (MQ4.112hz_3_2).

In some embodiments the antibody may comprise a set of CDRH1, CDRH2, CDRH3, CDRL1, CDRL2, CDRL3 amino acid sequences selected from (a) SEQ ID NOs: 35, 37, 39, 51, 53 and 55 (MQ4.112hz_3_1); (b) SEQ ID NOs: 19, 21, 23, 51, 53 and 55 (MQ4.112hz_1_1); and (d) SEQ ID NOs: 43, 45, 47, 51, 53 and 55 (MQ4.112hz_4_1). In some embodiments the antibody may comprise the HCVR and LCVR amino acid sequences of (a) SEQ ID NOs: 33 and 49; (MQ4.112hz_3_1); (b) SEQ ID NOs: 17 and 49 (MQ4.112hz_1_1); (c) SEQ ID NOs: 25 and 49 (MQ4.112hz_2_1); and (d) SEQ ID NOs: 41 and 49 (MQ4.112hz_4_1).

In some embodiments the antibody may comprise a set of CDRH1, CDRH2, CDRH3, CDRL1, CDRL2, CDRL3 amino acid sequences selected from (a) SEQ ID NOs: 35, 37, 39, 51, 53 and 55 (MQ4.112hz_3_1). In some embodiments the antibody may comprise the HCVR and LCVR amino acid sequences of (a) SEQ ID NOs: 33 and 49; (MQ4.112hz_3_1).

The CDRs of the heavy chain (CDRH) and light chain variable domain (CDRL) are located at residues 27-38 (CDR1), residues 56-65 (CDR2) and residues 105-117 (CDR3) of each chain according to the IMGT numbering system (http://www.imgt.org; Lefranc MP, 1997, J, Immunol. Today, 18, 509). This numbering system is used in the present specification except where otherwise indicated.

The antibody of the invention may comprise at least four, five, or all six CDRs, as listed in Table 2, of an antibody listed in Table 3 or any of the other groups of antibodies described herein. The antibody may comprise at least one, at least two, or all three heavy chain CDRs (CDRHs). The antibody may comprise at least one, at least two, or all three light chain CDRs (CDRLs). The antibody typically comprises all six (i.e. three heavy and three light chain) CDRs.

The antibody of the invention may comprise a heavy chain variable region having ≥80%, ≥85%, ≥90%, ≥95%, ≥96%, ≥97%, ≥98%, ≥99% or 100% sequence identity to the heavy chain variable region amino acid sequence of any of these antibodies.

The antibody of the invention may comprise a light chain variable region having ≥80%, ≥85%, ≥90%, ≥95%, ≥96%, ≥97%, ≥98%, ≥99% or 100% sequence identity to the light chain variable region amino acid sequence of any of these antibodies.

For example, in some embodiments, the antibody comprises (a) a heavy chain variable region having at least 80% amino acid sequence identity to any one of SEQ ID NOs: 1, 17, 25, 33 and 41, and/or SEQ ID NOs: 65, 81 and 89, or comprising the amino acid sequence of any one of SEQ ID NOs: 1, 17, 25, 33 and 41, and/or SEQ ID NOs: 65, 81 and 89; and/or (b) a light chain variable region having at least 80% amino acid sequence identity to any one of SEQ ID NOs: 1, 17, 25, 33 and 41, and/or SEQ ID NOs: 65, 81 and 89or comprising the amino acid sequence of any one of SEQ ID NOs: 1, 17, 25, 33 and 41, and/or SEQ ID NOs: 65, 81 and 89.

The antibody of the invention may comprise a heavy chain variable region having ≥80%, ≥85%, ≥90%, ≥95%, ≥96%, ≥97%, ≥98%, ≥99% or 100% sequence identity to the heavy chain variable region amino acid sequence of any of these antibodies and a light chain variable region having ≥80%, ≥85%, ≥90%, ≥95%, ≥96%, ≥97%, ≥98%, ≥99% or 100% sequence identity (or the same minimal percentage identity as defines the heavy chain variable region) to the matching light chain variable region amino acid sequence of the same antibody, as shown in Table 3.

Typically, the non-identical amino acids, as described above, of a variable region are not in the CDRs. Hence, an antibody of the invention may comprise 100% sequence identity over all six CDRs of a reference antibody from Table 3, but only ≥80%, ≥85%, ≥90%, ≥95%, ≥96%, ≥97%, ≥98%, ≥99% or 100% sequence identity over the complete length of the heavy and/or light chain variable regions of the same reference antibody.

An antibody of the invention may comprise a modification from the amino acid sequence of an antibody in Table 1, or any of the other groups of antibodies described herein, whilst maintaining the activity and/or function of the antibody. The modification may be a substitution, deletion and/or addition. For example, the modification may comprise 1, 2, 3, 4, 5, up to 10, up to 20, up to 30 or more amino acid substitutions and/or deletions from the amino acid sequence of the antibody of Table 1. For example, the modification may comprise an amino acid substituted with an alternative amino acid having similar properties. Some properties of the 20 main amino acids, which can be used to select suitable substituents, are as follows:

**Table 1**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged (+) | Trp | aromatic, hydrophobic, neutral |
| Leu | aliphatic, hydrophobic, neutral | Tyr | aromatic, polar, hydrophobic |

The modification may comprise a derivatised amino acid, e.g. a labelled or non-natural amino acid, providing the function of the antibody is not significantly adversely affected.

Modification of antibodies of the invention as described above may be prepared during synthesis of the antibody or by post-production modification, or when the antibody is in recombinant form using the known techniques of site-directed mutagenesis, random mutagenesis, or enzymatic cleavage and/or ligation of nucleic acids.

Antibodies of the invention may be modified (e.g. as described above) to improve the potency of said antibodies. Methods for identifying residues of an antibody that may be substituted are known in the art.

Antibodies of the invention may be isolated antibodies. An isolated antibody is an antibody which is substantially free of other antibodies having different antigenic specificities.

The term 'antibody' as used herein may relate to whole, full-length antibodies (i.e. comprising two antibody heavy chains and two light chains inter-connected by disulphide bonds), as well as antigen-binding fragments thereof. The term may also encompass VHH antibodies (heavy-chain antibodies), having two heavy chains only and no light chains, and antigen binding fragments thereof. Antibodies typically comprise immunologically active portions of immunoglobulin (Ig) molecules, i.e., molecules that contain an antigen binding site that specifically binds (immunoreacts with) an antigen. By "specifically binds" or "immunoreacts with" is meant that the antibody reacts with one or more antigenic determinants of an antigen, i.e. in the normal way that antibodies bind to antigen, forming an antibody-antigen complex. Each heavy chain is typically comprised of a heavy chain variable region (abbreviated herein as HCVR or VH) and at least one heavy chain constant region. Each light chain is typically comprised of a light chain variable region (abbreviated herein as LCVR or VL) and a light chain constant region. Typically the variable regions of the heavy and light chains contain a binding domain that interacts with an antigen, except for VHH antibodies where the variable regions of the heavy chains only comprise the antigen binding domain. The VH and VL regions can be further subdivided into regions of hypervariability, termed complementarity determining regions (CDR), interspersed with regions that are more conserved, termed framework regions (FR). Antibodies may include, but are not limited to, polyclonal, monoclonal, chimeric, recombinant, dAb (domain antibody), single chain, Fab (fragment antigen-binding regions), Fab' and F(ab')2 fragments, scFvs (single chain variable fragments), and Fab expression libraries.

An antibody of the invention may be a monoclonal antibody. Monoclonal antibodies (mAbs) of the invention may be produced by a variety of techniques, including conventional monoclonal antibody methodology, for example those disclosed in "Monoclonal Antibodies: a manual of techniques"(Zola H, 1987, CRC Press) and in "Monoclonal Hybridoma Antibodies: techniques and applications" (Hurrell JGR, 1982 CRC Press).

An antibody of the invention may be a multivalent antibody. "Multivalent antibody," as used herein, is an antibody comprising three or more antigen binding sites. The multivalent antibody may be engineered to have the three or more antigen binding sites. The antibodies of the present invention can be multivalent antibodies (which are other than of the IgM class) with three or more antigen binding sites (e.g. "trivalent", "trimeric", "tetravalent" or "tetrameric" antibodies). Such antibodies can be produced by recombinant expression of nucleic acid encoding the polypeptide chains of the antibody, or using methods described further below and known in the art. The multivalent antibody may comprise a dimerization domain and three or more antigen binding sites. The dimerization domain may comprise (or consists of) an Fc region or a hinge region. The antibody will then comprise an Fc region and three or more antigen binding sites amino-terminal to the Fc region. The multivalent antibody may comprise or consist of three to about eight antigen binding sites. The multivalent antibody comprises at least one polypeptide chain or may comprise two polypeptide chains, wherein the polypeptide chain(s) comprise two or more variable domains. For instance, the polypeptide chain(s) may comprise VDl-(Xl )n-VD2-(X2)n-Fc, wherein VD1 is a first variable domain, VD2 is a second variable domain, Fc is one polypeptide chain of an Fc region, XI and X2 represent an amino acid or polypeptide, and n is 0 or 1. For instance, the polypeptide chain(s) may comprise: VH-CH1-flexible linker-VH-CHl-Fc region chain; or VH-CH 1-VH-CH 1-Fc region chain. Or VH-CH1-CH2-CH3(Fc region)-Flexible linker1-VH-Flexible linker2-VL.The multivalent antibody may further comprises at least two, or four light chain variable domain polypeptides. The multivalent antibody may, for instance, comprise from about two to about eight light chain variable domain polypeptides. The light chain variable domain polypeptides may comprise a light chain variable domain and, optionally, further comprise a CL domain. The multivalent antibody may be IgG.

An antibody or multivalent antibody of the invention may be a multispecific antibody, for example, a bispecific antibody or a trispecific antibody. "Multispecific antibody" is an antibody having at least two different binding sites, each site with a different binding specificity. A multispecific antibody can be a full-length antibody or an antibody fragment, and the different binding sites may bind each to a different antigen or the different binding sites may bind to two different epitopes of the same antigen. In some cases a bispecific antibody of the invention may bind to two different antigens. One of the binding sites of the multispecific antibody is provided by the antibody specific for collagen type II (or antigen-binding fragment thereof). The other binding site(s) may be provided by another antibody (or antigen-binding fragment thereof) with different specificity, such as specificity for an antigen present in bone cells. In this context, the antibody may inhibit osteoclastogenesis. A specific example target in bone cells is RANKL, which is the target of the monoclonal antibody Denosumab. The multispecific antibody may thus include Denosumab or an antigen binding fragment thereof. Other targets include growth factors or growth factor receptors, or inflammatory cytokines or their receptors. The other binding site(s) may thus be from antibodies (or antigen binding fragments thereof) which bind to these targets, such as antibodies which bind to GM-CSF (e.g. otilimab (GSK3196165)), or GM-CSF receptor inhibitory antibodies (e.g. as Mavrilimumab). Alternatively, the other binding site(s) may be provided by any other form of binding moiety. For example, the other binding site(s) may itself be a growth factor, thereby providing binding specificity for the corresponding growth factor receptor or binding partner, or it may be an inflammatory cytokine, thereby providing binding specificity for the corresponding cytokine receptor or binding partner. Such embodiments may alternatively be described herein as molecules in which the antibody specific for collagen type II is linked, fused, or conjugated to another therapeutic agent. Examples of suitable growth factors include nerve growth factor (NGF), TGF-β1 = transforming growth factor-β1; BMP-2 and BMP-7 = bone morphogenetic protein; IGF-I = insulin growth factor I; FGF = fibroblast growth factor (including FGF18 and derivatives and truncated versions thereof, e.g. sprifermin); PDGF = platelet-derived growth factor. Examples of suitable cytokines include (IL)-1, IL-4, IL-6, IL-10, IL-11, IL-13, TGF-β and TNF- α, in particular IL-1 and IL-6 and TNF-α. Other targets for treating OA or RA, as discussed elsewhere herein, are also contemplated.

Techniques for making multispecific and multivalent antibodies include, but are not limited to, recombinant co-expression of two immunoglobulin heavy chain-light chain pairs having different specificities (see e.g., Milstein and Cuello, Nature 305: 537 (1983), WO 93/08829, and Traunecker et al., EMBOJ. 10: 3655 (1991)). "Knob-in-hole" engineering can also be used (see, e.g., U.S. Patent No. 5,731,168). In this method, "knobs" are constructed by replacing small amino acid side chains from the interface of a first polynucleotide with larger side chains (e.g. tyrosine or tryptophan). Complementary "holes" of identical or similar size to the "knobs" are optionally created on the interface of the second polynucleotide by replacing large amino acid chains with smaller ones (e.g. alanine or threonine). Where a suitably positioned and dimensioned "knob" or "hole" exists at the interface of either the first or the second polynucleotide, it is only necessary to engineer a corresponding "hole" or "knob", respectively, at the adjacent interface. Accordingly, a multispecific or multivalent antibody of the invention may comprise a first polynucleotide and a second polynucleotide which meet at an interface, wherein the first polynucleotide has a "knob" at the interface thereof which is positionable in a "hole" at the interface of the second polynucleotide.

Multispecific antibodies can also be made by engineering "electrostatic steering" effects that favor formation of Fc-heterodimeric antibody molecules rather than homodimers (WO 2009/089004A1); cross-linking two or more antibodies or fragments (see, e.g., US Pat. No. 4,676,980, and Brennan et al., Science, 229: 81 (1985)); using leucine zippers to produce bispecific antibodies (see, e.g., Kostelny et al., J. Immunol, 148(5): 1547-1553 (1992)); using "diabody" technology for making bispecific antibody fragments (see, e.g., Hollinger et al., Proc. Natl. Acad. Sci. USA, 90:6444-6448 (1993)); using single-chain Fv (scFv) dimers (see, e.g. Gruber et al., J. Immunol, 152:5368 (1994)); or tri-specific antibodies (see e.g., Tutt et al., J. Immunol. 147: 60 (1991).

Multispecific antibodies of the invention can also be made by fusing a classical antibody to the C-terminus of an ScFv antibody.

It is contemplated that any of the antibodies of the present invention described herein can be prepared as multispecific antibodies using the methods and techniques known in the art and/or described herein.

A multispecific antibody of the present invention may comprise an hIgG Fc region. The hIgG region may be a LaLaNa region or variant thereof. The constant region domains of an antibody molecule of the invention, if present, may be selected having regard to the proposed function of the antibody molecule, and in particular the effector functions which may be required. For example, the constant region domains may be human IgA, IgD, IgE, IgG or IgM domains. In particular, human IgG constant region domains may be used, especially of the IgG1 and IgG3 isotypes when the antibody molecule is intended for therapeutic uses where antibody effector functions are required. Alternatively, IgG2 and IgG4 isotypes may be used when the antibody molecule is intended for therapeutic purposes and antibody effector functions are not required. Typically, the constant regions are of human origin. In particular, human IgG (i.e. IgG1, IgG2, IgG3 or IgG4) constant region domains may be used. Most typically, a human IgG1 constant region is used, or mutant variant thereof that lack effector functions, such as a variant having the VH domain LaLaNa mutation of the human Fc of IgG1.

The light chain constant region may be either lambda or kappa.

An antibody of the invention may be a chimeric antibody, a CDR-grafted antibody, a nanobody, a human antibody or humanised antibody. Typically, the antibody is a human or a humanized antibody. Fully human antibodies are those antibodies in which the variable regions and the constant regions (where present) of both the heavy and the light chains are all of human origin, or substantially identical to sequences of human origin, but not necessarily from the same antibody.

The antibody of the invention may be an antigen-binding fragment. An antigen-binding fragment of the invention binds to the same epitope as a reference full length antibody or parent antibody, i.e. the antibody from which the antigen-binding fragment is derived. An antigen-binding fragment of the invention typically retains the parts of the parent antibody that interact with the epitope. Typically, the antigen-binding fragment retains the same or similar binding affinity to the antigen as the reference antibody. Methods for creating and manufacturing antibody fragments are well known in the art (see for example Verma R et al., 1998, J. Immunol. Methods, 216, 165-181).

An antigen-binding fragment does not necessarily have an identical sequence to the parent antibody. In one embodiment, the antigen-binding fragment may have ≥70%, ≥80%, ≥85%, ≥90%, ≥95%, ≥96%, ≥97%, ≥98%, ≥99%, 100% sequence identity with the respective variable region domains of the parent antibody. Typically, the non-identical amino acids of a variable region are not in the CDRs.

Methods for screening antibodies of the invention that do not share 100% amino acid sequence identity with one of the antibodies disclosed herein, that possess the desired specificity, affinity and functional activity include enzyme linked immunosorbent assays, biacore, focus reduction neutralisation assay (FRNT), and other techniques known within the art or described herein.

An antibody of the invention retains the ability to bind to collagen type II and to distinguish between damaged and undamaged cartilage as described herein. For example, any one of the relevant methods described herein or in the Examples herein may be used to determine this property.

An antibody of the invention may have an affinity constant (K_{D}) value for collagen type II of ≤5nM, ≤4nM, ≤3nM, ≤2nM, ≤1nM, ≤0.5nM, ≤0.4nM, ≤0.3nM, ≤0.2nM, ≤0.1nM or ≤0.05nM.

The KD value can be measured by any suitable means known in the art, for example, by ELISA or Surface Plasmon Resonance (Biacore) at 25 °C.

Antibodies of the invention may have any combination of one or more of the properties described herein.

Antibodies of the invention may bind to the same epitope as, or compete for binding to collagen type II with, any one of the antibodies described herein (i.e. in particular with antibodies having the CDR sequences or the heavy and light chain variable regions described herein).

The skilled person is able to determine the binding site (epitope) of an antibody, whether an antibody binds to the same epitope as, or competes for binding with, an antibody described herein, using techniques known in the art.

For example, to determine if a test antibody binds to the same epitope as an antibody described herein (referred to a "reference antibody" in the following paragraphs), the reference antibody may be allowed to bind to a protein or peptide under saturating conditions. Next, the ability of a test antibody to bind to the protein or peptide is assessed. If the test antibody is able to bind to the protein or peptide following saturation binding with the reference antibody, it can be concluded that the test antibody binds to a different epitope than the reference antibody. On the other hand, if the test antibody is not able to bind to protein or peptide following saturation binding with the reference antibody, then the test antibody may bind to the same epitope as the epitope bound by the reference antibody of the invention.

To determine if an antibody competes for binding with a reference antibody, the above-described binding methodology is performed in two orientations. In a first orientation, the reference antibody is allowed to bind to a protein/peptide under saturating conditions followed by assessment of binding of the test antibody to the protein/peptide molecule. In a second orientation, the test antibody is allowed to bind to the protein/peptide under saturating conditions followed by assessment of binding of the reference antibody to the protein/peptide. If, in both orientations, only the first (saturating) antibody is capable of binding to the protein/peptide, then it is concluded that the test antibody and the reference antibody compete for binding to the protein/peptide. As will be appreciated by the skilled person, an antibody that competes for binding with a reference antibody may not necessarily bind to the identical epitope as the reference antibody, but may sterically block binding of the reference antibody by binding an overlapping or adjacent epitope.

Two antibodies bind to the same or overlapping epitope if each competitively inhibits (blocks) binding of the other to the antigen. Alternatively, two antibodies have the same epitope if essentially all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Additional routine experimentation (e.g., peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

As well as sequences defined by percentage identity or number of sequence changes, the invention further provides an antibody defined by its ability to cross-compete with one of the specific antibodies set out herein. It may be that the antibody also has one of the recited levels of sequence identity or number of sequence changes as well.

Cross-competing antibodies can be identified using any suitable method in the art, for example by using competition ELISA or BIAcore assays where binding of the cross competing antibody to a particular epitope on collagen type II prevents the binding of an antibody of the invention or *vice versa.* In one embodiment, the antibody produces ≥50%, ≥60%, >_70%, ≥80%, ≥90% or 100% reduction of binding of the specific antibody disclosed herein.

Appropriate antibodies described below in the Examples may be used as reference antibodies.

Other techniques that may be used to determine antibody epitopes include hydrogen/deuterium exchange, X-ray crystallography and peptide display libraries. A combination of these techniques may be used to determine the epitope of the test antibody.

The approaches described herein could be applied equally to other data, e.g. surface plasmon resonance or ELISA, and provides a general way of rapidly determining locations from highly redundant competition experiments.

### Polynucleotides, vectors and host cells

The invention provides one or more isolated polynucleotides (e.g. DNA or RNA) encoding an antibody of the invention. In some cases, the complete sequence encoding the complete antibody may be spread across more than one polynucleotide, but the polynucleotides combined are able to encode an antibody of the invention. For example, the polynucleotides may encode the heavy and/or light chain variable regions(s) of an antibody of the invention. The polynucleotides may encode the full heavy and/or light chain of an antibody of the invention. Typically, one polynucleotide would encode each of the heavy and light chains.

The skilled person is readily able to use the amino acid sequences of other antibodies disclosed herein, to arrive at suitable polynucleotides encoding those antibodies.

Polynucleotides which encode an antibody of the invention can be obtained by methods well known to those skilled in the art. For example, DNA sequences coding for part or all of the antibody heavy and light chains may be synthesised as desired from the corresponding amino acid sequences.

General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

A polynucleotide of the invention may be provided in the form of an expression cassette, which includes control sequences operably linked to the inserted sequence, thus allowing for expression of the antibody of the invention *in vivo.* Hence, the invention also provides one or more expression cassettes encoding the one or more polynucleotides that encode an antibody of the invention. These expression cassettes, in turn, are typically provided within vectors (e.g. plasmids or recombinant viral vectors). Hence, in one embodiment, the invention provides a vector encoding an antibody of the invention. In another embodiment, the invention provides vectors which collectively encode an antibody of the invention. The vectors may be cloning vectors or expression vectors. A suitable vector may be any vector which is capable of carrying a sufficient amount of genetic information, and allowing expression of a polypeptide of the invention.

The polynucleotides, expression cassettes or vectors of the invention are introduced into a host cell, e.g. by transfection. Hence, the invention also provides a host cell comprising the one or more polynucleotides, expression cassettes or vectors of the invention. The polynucleotides, expression cassettes or vectors of the invention may be introduced transiently or permanently into the host cell, allowing expression of an antibody from the one or more polynucleotides, expression cassettes or vectors. Such host cells include transient, or preferably stable higher eukaryotic cell lines, such as mammalian cells or insect cells, lower eukaryotic cells, such as yeast, or prokaryotic cells, such as bacteria cells. Particular examples of cells include mammalian HEK293, such as HEK293F, HEK293T, HEK293S or HEK Expi293F, CHO, HeLa, NS0 and COS cells, or any other cell line used herein, such as the ones used in the Examples. Preferably the cell line selected will be one which is not only stable, but also allows for mature glycosylation.

The invention also provides a process for the production of an antibody of the invention, comprising culturing a host cell containing one or more vectors of the invention under conditions suitable for the expression of the antibody from the one or more polynucleotides of the invention, and isolating the antibody from said culture.

### Detectable Labels and Therapeutic Agents

In particular embodiments, the antibody or binding fragment thereof of the invention may be linked (directly or indirectly) to another moiety. The other moiety may be a therapeutic agent as described herein, such as a drug. Linking the drug to the antibody enables the drug to be targeted to and concentrate at the site of cartilage damage and localise the treatment. The other moiety may be a detectable label, which allows for monitoring of antibody binding to target and the visualisation, detection or quantification of cartilage damage. The other moiety may be a binding moiety, such as an antigen binding domain specific for a therapeutic target, e.g. the second arm of a bispecific antibody as described herein. The other moiety may be an (immuno-)liposome which may contain a therapeutic agent and/or a detectable label.

By "detectable label" it is meant that the antibody is linked to a moiety which, when located at the target site following administration of the antibody to a subject, may be detected, typically non-invasively from outside the body and the site of the target located. Thus, the antibody may be useful in imaging and diagnosis.

In some cases the detectable label is or comprises a fluorescent moiety (probe) or a bioluminescent moiety. Fluorescent moieties include far-red (600 to 700 nm wavelength) fluorophores, such as Cy5.5 and small ultra red fluorescent protein (smURFP), and near infrared (NIR, 700 to 1700 nm, or more typically, 700 to 900 nm wavelength) fluorophores, such as polymethine cyanines (for example indocyanine green (ICG) and derivatives thereof such as hydrophilic glucamide-derivatized indocyanine and PPCy dyes), phthalocyanines, squaraines, BODIPY (borondipyrromethane) dyes, tetrapyrrolebased compounds (for example hematoporphyrin (HpD), meso-tetra-m-hydroxyphenylchlorin (m-THPC) benzoporphyrin derivatives (BPD), and sulfonated phthalocyanines) and derivatives thereof. Other specific examples include cypate, ProSense750, Perkin Elmer; and IRDye^{®} 800CW 2-DG, LiCor Biosciences. The use of antibodies linked to quantum dots, quantum dot nanoparticles or nanoparticles comprising NIR dye encapsulated by a polymer or matrix-based coat (for example as described elsewhere herein in relation to immunoliposomes) are also contemplated.

Fluorescent moieties that are activated by protease activity may be used. These include probes that are activated by Cathespins or matric metalloproteinases (MMPs), which are particularly suitable.

In some cases the detectable label is or comprises a radioactive atom or radioisotope. Such a label may be detected by, and the methods described herein may comprise, (immune)scintiography or single photon emission computer tomography (SPECT). Suitable radioactive atoms/radioisotopes include 99mTc, 123I and 111In. Other labels include, for example, spin labels for magnetic resonance imaging (MRI) such as 123I again, 1311, 111In, 19F, 13C, 15N, 170, gadolinium, manganese or iron. The radioor other labels may be incorporated in known ways. For example, the antibody, or fragment thereof, may be biosynthesised or may be synthesised by chemical amino acid synthesis using suitable amino acid precursors involving, for example, fluorine-19 in place of hydrogen. Labels such as 99mTc, 123I, 186Rh, 188Rh and 111In can, for example, be attached via cysteine residues in polypeptides. 123I, 186Rh, 188Rh and 111In Yttrium-90 can be attached via a lysine residue. Most typically, the detectable label comprises a radioactive atom, such as, for example technetium-99m or iodine-123. Alternatively, the detectable label may be selected from the group comprising: iodine-123; iodine-131; indium-111; fluorine-19; carbon-13; nitrogen-15; oxygen-17; gadolinium; manganese; and iron.

In some cases the antibodies may be used for immuno-positron emission tomography (PET). Hence, the antibody may be linked, fused or conjugated to a positron emitter and/or a chelating moiety. The methods described herein may comprise detecting, imaging visualising and/or quantitating cartilage damage or loss of cartilage proteoglycan by (immuno-)PET. Suitable methods are described in The Oncologist, 12: 1379 (2007); Journal of Nuclear Medicine, 52(8): 1171 (2011). Exemplary positron emitters include ⁸⁹Zr, ⁶⁸Ga, ⁶⁴Cu, ⁴⁴Sc, ⁸⁶Y, ⁷⁶Br, ¹²⁴I, and ¹⁸F. Most typically, the positron emitter is ¹⁸F . The positron emitter may form a stable complex with the chelating moiety and have physical half-lives suitable for immuno-PET imaging purposes. The chelating moiety comprises a portion capable of chelating a positron emitter (reacts with a positron emitter to form a coordinated chelate complex). Exemplary chelating moieties are described in Nature Protocols, 5(4): 739, 2010; Bioconjugate Chem., 26(12): 2579 (2015); Chem Commun (Camb), 51(12): 2301 (2015); Mol. Pharmaceutics, 12: 2142 (2015); Mol. Imaging Biol., 18: 344 (2015); Eur. J. Nucl. Med. Mol. Imaging, 37:250 (2010); Eur. J. Nucl. Med. Mol. Imaging (2016); and Bioconjugate Chem., 26(12): 2579 (2015) and include those that comprise desferrioxamine (DFO), 1,4,7,10-tetraacetic acid (DOTA), diethylenetriaminepentaacetic acid (DTPA), ethylenediaminetetraacetic acid (EDTA), (1,4,7,10-Tetraazacyclododecane-1,4,7,10-tetra(methylene phosphonic) acid (DOTP), DOTMA, 1,4,8,11-Tetraazacyclotetradecane-1,4,8, 11-tetraacetic acid (TETA), H₄octapa, H₆phospa, Hzdedpa, Hsdecapa, H₂azapa, HOPO, DO2A, 1,4,7,10-Tetrakis(carbamoylmethyl)-1,4,7,10-tetraazacyclododecane (DOTAM), 1,4,7-triazacyclononane-N,N',N"-triacetic acid (NOTA), 1,4,7,10-Tetrakis(carbamoylmethyl)-1,4,7,10-tetraazacyclododecane (DOTAM), 1,4,8,11-tetraazabicyclo[6.6.2]hexadecane-4, 11-dicetic acid (CB-TE2A), 1,4,7,10-Tetraazacyclododecane (Cyclen), 1,4,8,11-Tetraazacyclotetradecane (Cyclam), octadentate chelators, hexadentate chelators, phosphonate-based chelators, macrocyclic chelators, chelators comprising macrocyclic terephthalamide ligands, bifunctional chelators, fusarinine C and fusarinine C derivative chelators, triacetylfusarinine C (TAFC), ferrioxamine E (FOXE), ferrioxamine B (FOXB) and ferrichrome A (FCHA).

In some cases the antibody may be linked, fused or conjugated to a therapeutic agent or to a carrier for a therapeutic agent to treat OA or RA or to prevent or reduce cartilage damage or loss of cartilage proteoglycan. In some cases a cleavable linker may be used. Typically, the linker is or comprises a polymer. The linker may be a peptide linker. The amino acids in the linker may be directly attached e.g. by peptide bonds, or may be separated by one or more chemical spacers such as an OC(O) group. Typically, the linker comprises a feature which allows it to be selectively cleaved in the vicinity of cartilage or damaged cartilage. The linker may comprise a protease recognition site. The site may be capable of being cleaved by a protease expressed at the target site. Metalloproteinases are implicated in the pathogenic degradation of collagen type II in arthritis and may therefore be localized to sites of cartilage damage. Hence, the linker may be a metalloproteinase or a matrix-metalloproteinase (MMP) cleavable linker.

The therapeutic agent linked to the antibody may comprise a polypeptide or a polynucleotide encoding a polypeptide which is of therapeutic benefit. Examples of such polypeptides include anti-proliferative cytokines, anti-inflammatory cytokines, or growth factors. Example of cytokines include interleukin (II,)-1, IL-4, IL-6, IL-10, IL-11, IL-13 and (TGF)-β. Examples of growth factors include nerve growth factor (NGF), TGF-β1 = transforming growth factor-β1; BMP-2 and BMP-7 = bone morphogenetic protein; IGF-I = insulin growth factor I; FGF = fibroblast growth factor (including FGF18 and derivatives and truncated versions thereof, e.g. sprifermin); PDGF = platelet-derived growth factor. FGF18 and derivatives and truncated versions thereof, such as sprifermin and bone morphogenetic protein 7 (BMP7, also known as osteogenic protein-1 or OP-1) are preferred.

In some cases the antibody is linked, conjugated or fused to a superparamagnetic iron oxide nanoparticle (SPION), as described, for example, in In. J. Nanomedicine: 3445-3471 (2022). SPIONs are typically small synthetic γ-Fe2O3 (maghemite), Fe3O4(magnetite) or α-Fe2O3 (hermatite) particles with a core typically ranging from 10 nm to 100 nm in diameter and a biocompatible polymers, such as dextran, polyethylene glycol, amorphous silica or calcium phosphate. In some cases, PLGA/polylactic-cogylcolic acid is a suitable coating because it has particular biocompatibility with synovial tissues and/or for intra-articular treatment. SPIONs may be used for either magnetic resonance imaging, where they act as magnetic resonance contrast agents, or as a delivery vehicle for therapeutic agents. A therapeutic agent may be loaded on/conjugated to the coat (directly or via a linker, as described herein) or enclosed/encapsulated within the coat or within a phospholipid bilayer (magnetopliposomes) together with the magnetic core. Specific examples are SPION-corticosteroid conjugates and coencapsulated dexamethasone acetate. The particles may be guided to their target tissue using an external magnet (in addition to targeting via the antibodies).

In some cases the antibody is conjugated to an (immuno-)liposome. The immunoliposome may encapsulate a therapeutic agent, as described herein, including for example anti-proliferative cytokines, anti-inflammatory cytokines, or growth factors as described herein.
In specific examples, the immunoliposome may contain a derivative of fibroblast growth factor 18 (FGF18) or a therapeutically effective truncated form thereof, such as Sprifermin. The immunoliposome may contain an anti-inflammatory cytokine, such as interleukin (IL)-1, IL-4, IL-6, IL-10, IL-11, IL-13 and (TGF)-β. The immunoliposome may contain bone morphogenetic protein 7 (BMP7, also known as osteogenic protein-1 or OP-1). In other examples, the immunoliposome may contain an inhibitor of an MMPs (such as MMP-13, MMP-1 and MMP-7) or aggrecanase (such as ADAMTS-4 nd -5) (such as PG-116800 (PG-530742), CP-544439, AZD-8955, and WAY-170523); a cathepsin K inhibitor (such as MIV-711); a bisphosphonate; a TGFβ inhibitor; Teriparatide; a non-steroidal anti-inflammatory drug (such as naproxen); a glucocorticoids; hydroxychloroquine; an I-κB kinase inhibitor (such as SAR113945); a p38 MAPK inhibitor (such as PH-797804 and FX-005); or a TLR7 inhibitor (such as miR-21).

The immunoliposomes may typically be about 70 to 300 nm, or 150 to 250 nm in diameter. In some cases, the immunoliposomes may be formulated using PEGylated phospholipids. In more specific cases, they may be Type C PEG-immunoliposomes, as described, for example, in Maruyama et al. Bioci Rep. 2002 22(2): 251-66. The antibodies may be linked via PEG-malemide at the distal end of the PEG chains. Specific examples of PEG lipids include 1,2-Distearoyl-sn-glycero-3-phosphoethanolamine-N-(amino (polyethylene glycol)-2000) (DSPE-PEG(2000)); 1,2-Distearoyl-sn-glycero-3-phosphoethanolamine-N-(amino (polyethylene glycol)-5000)(DSPE-PEG(5000)), DMPE-((polyethylene glycol)-2000)(C14-PEG), 1,2-Dimyristoyl-rac-glycero-3-methoxypolyethylene glycol-2000
(DMG-PEG(2000)). Other suitable immunoliposomes and their preparation are described in Fobian et al. Pharmaceutics. 2021 Dec 23;14(1):26.

The linker or moiety may be directly attached, for example by chemical conjugation. Methods of conjugating agents or labels to an antibody are known in the art. For example, carbodiimide conjugation (Bauminger S and Wilchek M, 1980, Methods Enzymol., 70, 151-159) may be used to conjugate a variety of agents to antibodies or peptides. The water-soluble carbodiimide, 1-ethyl-3-(3-dimethylaminopropyl) carbodiimide (EDC) is particularly useful for conjugating a functional moiety to a binding moiety. Other methods for conjugating a moiety to antibodies can also be used. For example, sodium periodate oxidation followed by reductive alkylation of appropriate reactants can be used, as can glutaraldehyde cross-linking. The linker or other moiety, as described above typically comprises a terminal natural or non-natural amino acid having a reactive side chain capable of binding to a reactive functional group on the antibody, for example a group Caa as described above. The terminal group may for example be a cysteine or a lysine residue. Any suitable attachment chemistry can be used. For example, cysteine residues can be used to react with a mameimide group or form disulphide bonds, or a cysteine residue may be linked to a lysine residue using a amine-to-sulfhydryl crosslinker such as MBS (*m*-maleimidobenzoyl-N-hydroxysuccinimide ester). Many other suitable reactive groups and their chemical targets are known in the art. Some exemplary reactive groups and their corresponding targets include aryl azides which may react with amine, carbodiimides which may react with amines and carboxyl groups, hydrazides which may react with carbohydrates, hydroxmethyl phosphines which may react with amines, imidoesters which may react with amines, isocyanates which may react with hydroxyl groups, carbonyls which may react with hydrazines, maleimides which may react with sulfhydryl groups, NHS-esters which may react with amines, PFP-esters which may react with amines, psoralens which may react with thymine, pyridyl disulfides which may react with sulfhydryl groups, vinyl sulfones which may react with sulfhydryl amines and hydroxyl groups, vinylsulfonamides, and the like. Suitable click chemistry reagents are known in the art, for example, copper(I)-catalyzed azide-alkyne cycloadditions (azide alkyne Huisgen cycloadditions); strain-promoted azide-alkyne cycloadditions; including alkene and azide [3+2] cycloadditions; alkene and tetrazine inverse-demand Diels-Alder reactions; and alkene and tetrazole photoclick reactions; copper-free variant of the 1,3 dipolar cycloaddition reaction, where an azide reacts with an alkyne under strain, for example in a cyclooctane ring such as in bicycle[6.1.0]nonyne (BCN); the reaction of an oxygen nucleophile on one linker with an epoxide or aziridine reactive moiety on the other; and the Staudinger ligation, where the alkyne moiety can be replaced by an aryl phosphine, resulting in a specific reaction with the azide to give an amide bond. The linker or moiety may be conjugated N-terminal or C-terminal to the antibody.

### Pharmaceutical composition

The invention provides a pharmaceutical composition comprising an antibody of the invention. The pharmaceutical compositions or kits described herein may comprise, in addition to one or more antibodies, a pharmaceutically acceptable excipient, carrier, diluent, buffer, stabiliser, preservative, adjuvant or other materials well known to those skilled in the art. Such materials are preferably non-toxic and preferably do not interfere with the pharmaceutical activity of the active ingredient(s). Examples of suitable aqueous carriers include water, buffered water and saline. The composition of the invention may include one or more pharmaceutically acceptable salts. A "pharmaceutically acceptable salt" refers to a salt that retains the desired biological activity of the parent compound and does not impart any undesired toxicological effects. Examples of such salts include acid addition salts and base addition salts. Other suitable pharmaceutically acceptable carriers include ethanol, polyols (such as glycerol, propylene glycol, polyethylene glycol, and the like), and suitable mixtures thereof, vegetable oils, such as olive oil, and injectable organic esters, such as ethyl oleate. In many cases, it will be desirable to include isotonic agents, for example, sugars, polyalcohols such as mannitol, sorbitol, or sodium chloride in the composition. The precise nature of the carrier or other material may depend on the route of administration.

Therapeutic compositions are typically sterile and stable under the conditions of manufacture and storage. The composition can be formulated as a solution, microemulsion, liposome, or other ordered structure suitable to high drug concentration.

The pharmaceutical compositions of the invention may be co-formulated with, or be intended for administration with, one or more additional therapeutic agents, for example an anti-inflammatory agent.

The pharmaceutical composition may be formulated for subcutaneous, intravenous, intradermal, intramuscular, intranasal or oral administration. Administration may be systemic or intra-articular. An advantage of linking a therapeutic agent for OA or RA to an antibody of the invention is that a higher concentration of the drug can be achieved at the target site following systemic administration.

Also within the scope of the invention are kits comprising antibodies or other compositions of the invention and instructions for use, for example in any method of the invention. The kit may further contain one or more additional reagents, such as an additional therapeutic or prophylactic agent, for example as discussed herein.

### Therapeutic Uses

The term "treatment" as used herein includes therapeutic and prophylactic treatment. Administration is typically in a "prophylactically effective amount" or a "therapeutically effective amount" (although prophylaxis may be considered therapy/treatment), this being sufficient to result in a clinical response or to show clinical benefit to the individual, e.g. an effective amount to prevent or delay the onset of disease or any symptom thereof, to ameliorate one or more symptoms, to induce or prolong remission, or to delay relapse or recurrence. The methods and uses of the invention may comprise inhibiting the disease state, e.g. arresting its development; and/or relieving the disease state, e.g. causing regression of the disease state, e.g. until a desired endpoint is reached. The methods and uses of the invention may comprise the amelioration or the reduction of the severity, duration or frequency of a symptom of the disease state (e.g. lessen pain, discomfort, inflammation or immobility). Symptoms that may be affected by treatment may include joint pain, join stiffness, problems moving a joint, swelling, tenderness, inflammation. The most commonly affected joints are knees, hips and hand or foot joints. More general OA or RA-induced symptoms may include tiredness, an elevated temperature, sweating, loss of appetite and weight loss.

The invention provides a method of formulating a composition for treating arthritis, as described herein, or a disease or complication associated therewith, wherein said method comprises mixing an antibody or a pharmaceutical composition according to the invention with an acceptable carrier to prepare said composition.

Typically, the invention relates to methods and uses for a human subject in need thereof. However, non-human animals such as mice, rats, rabbits, sheep, pigs, cows, cats, or dogs is also contemplated.

The subject may be asymptomatic or pre-symptomatic. The subject may be early, middle or late phase of the disease. However, a particular advantage provided by the invention is the prospect for early detection of OA or RA. The subject may be male or female.

Antibodies or pharmaceutical compositions may be administered subcutaneously, intravenously, intradermally, intramuscularly, intranasally or orally. Most typically administration is systemic or intra-articular, as described herein. The pharmaceutical composition may be formulated for subcutaneous, intravenous, intradermal, intramuscular, intranasal or oral administration. Administration may be systemic or intra-articular.

The dose of an antibody may vary depending on the age and size of a subject, as well as on the disease, conditions and route of administration. Antibodies may be administered at a dose of about 0.1 mg/kg body weight to a dose of about 100 mg/kg body weight, such as at a dose of about 5 mg/kg to about 10 mg/kg. Antibodies may also be administered at a dose of about 50 mg/kg, 10 mg/kg or about 5 mg/kg body weight.

The antibody of the invention may be administered in a multiple dosage regimen. For example, the initial dose may be followed by administration of a second or plurality of subsequent doses. The second and subsequent doses may be separated by an appropriate time.

### Detecting, Quantifying and/or Imaging Cartilage Damage

Antibodies of the invention, may be used to detect, visualize, image and quantify cartilage damage and/or loss of cartilage proteoglycan, particularly damage/proteoglycan loss due to OA or RA. Hence, the antibodies and methods may be used to diagnose OA or RA, to monitor disease progression or regression, to determine a prognosis, to characterise the disease state, and monitor response to treatment. The method involves detecting, quantifying or imaging the presence of the antibody bound to the cartilage in an antibody-antigen complex. The methods may be particularly useful for early diagnosis or monitoring the early stages of OA or RA in a subject, e.g. within the first one or two years of disease onset.

The methods may be performed *in vivo* or *ex vivo* (e.g. on tissue or cartilage samples). In some cases, the method comprises the step of obtaining a suitable tissue or cartilage sample from a subject. The methods comprise contacting cartilage with an antibody of the invention. For *in vivo* detection, the antibody may be administered to the subject, for example as described elsewhere herein in relation to therapeutic uses. The methods further comprise detecting, quantifying or imaging the presence or absence of the antibody co-localised with, and hence bound to damaged cartilage. Hence, the method involves detecting, quantifying or imaging the presence of the antibody bound to the cartilage in an antibody-antigen complex. Binding of the antibody to cartilage may be detected by any suitable means, as known to those skilled in the art, and dependent on the antibody characteristics, particularly the identity of any detectable label that is used. The method may, for example, involve detecting an emitted fluorescent, radioactive or magnetic resonance signal. Other methods that are suitable, in particular, for ex vivo/in vitro methods, include include enzyme linked immunosorbent assays (ELISAs), Western blots, immunoprecipitations. The detection techniques may provide a qualitative or a quantitative readout depending on the assay employed.

The presence of antibody bound to cartilage (or of antibody-antigen complex), or increased antibody bound to the cartilage (antibody-antigen complex) compared to a reference (for example a reference corresponding to a subject/patient without OA/RA), indicates cartilage damage or loss of cartilage proteoglycan. Detection of increased antibody may inform a diagnosis of OA or RA, or a poor prognosis, may indicate disease progression or a poor response to a treatment, or may indicate the need for a treatment, a more aggressive treatment, a change of treatment, or an increased dose, frequency or intensity of a treatment. Decreased antibody detection compared to a reference may indicate a null diagnosis (no OA/RA diagnosis), a positive prognosis, indicate an improved disease state, remission, regression, or a positive response to a treatment, or indicate a less aggressive treatment, continued use of a treatment, or a decreased dose, frequency or intensity of a treatment. Hence, the method may be a method of determining the severity or progression of OA or RA in a subject.

In some embodiments, the method/detection may be repeated at two or more different time points. The level of detected antibody, the quantification, or the images obtained at each time point may be compared and used for diagnosis, prognosis, monitoring or assessing response to a treatment, as described herein. Hence, in some cases, the "reference" refers to data obtained from the same subject but at a different time point. In some embodiments the method is for monitoring the response of a subject to a particular treatment and the antibody is detected before and after (or during) treatment. The term "after treatment" means after the commencement of treatment and a period of time within which a response to treatment is expected. Hence, "after treatment" encompasses "during treatment", meaning that treatment may still be ongoing. Reduced antibody binding to cartilage after treatment indicates a positive response to treatment. A positive response to treatment may also be indicated by a stable/equivalent level of antibody binding to cartilage before and after treatment, or by increased binding of antibody to cartilage, in circumstances where disease progression and increased binding, or a greater extent of progression and antibody binding to cartilage is expected in the absence of the treatment. In some embodiments, the antibody and/or methods may be used for screening of therapeutic agents for treating OA or RA.

### Kits

The invention also encompasses kits for detecting cartilage damage or loss of cartilage proteoglycan or for carrying out any of the methods of the invention described herein. The kit comprises an antibody of the invention. The kit may further comprise other means and reagents for carrying out the methods. The kit may comprise a standard of reference sample. The kit components may be packaged in a suitable container. The kit may comprise instructions for using the kit or for carrying out the methods.

### Other

The present disclosure includes the combination of the aspects and features described except where such a combination is clearly impermissible or is stated to be expressly avoided. As used in this specification and the claims, the singular forms "a", "an", and "the" include plural referents unless the content clearly dictates otherwise. Thus, for example, reference to "an antibody" includes two or more such antibodies.

Section headings are used herein for convenience only and are not to be construed as limiting in any way.

When referring to "≥*x*" herein, this means equal to or greater than x. When referred to "≤*x*" herein, this means less than or equal to x.

For the purpose of this invention, in order to determine the percent identity of two sequences (such as two antibody sequences), the sequences are aligned for optimal comparison purposes (*e.g*., gaps can be introduced in a first sequence for optimal alignment with a second sequence). The amino acids at each position are then compared. When a position in the first sequence is occupied by the same amino acid as the corresponding position in the second sequence, then the amino acids are identical at that position. The percent identity between the two sequences is a function of the number of identical positions shared by the sequences (*i.e.*, % identity = number of identical positions /total number of positions in the reference sequence x 100).

Typically the sequence comparison is carried out over the length of the reference sequence. If the sequence is shorter than the reference sequence, the gaps or missing positions should be considered to be non-identical positions.

The skilled person is aware of different computer programs that are available to determine the homology or identity between two sequences using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6. Other examples of suitable programs are the BESTFIT program provided by the UWGCG Package (for example used on its default settings) (Devereux et al (1984) Nucleic Acids Research 12, 387-395) and the PILEUP and BLAST algorithms c (for example used on its default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (http://www.ncbi.nlm.nih.gov/).

The amino acid position numberings provided herein used the IMGT numbering system), although in some instances the KABAT numbering system or the absolute numbering of the amino acids based on the sequence listing may be used.

All publications, patents and patent applications cited herein, whether supra or infra, are hereby incorporated by reference in their entirety.

The following examples illustrate the invention.

### Examples

### Example 1

A panel of murine monoclonal antibodies were developed against bovine collagen type II. Bovine cartilage explants were damaged using 2.5% trypsin and subsequently incubated with a subset of eight different anti-collagen type II antibodies labeled with ¹²⁵I. Binding of 3 out of 8 antibodies to the damaged cartilage was significantly higher compared to binding of the isotype control antibody, namely antibodies 6 (p<0.01), 7 (p<0.0001) and 8 (p<0.0001) (Figure 1). Because of their high binding affinity, Ab7 (MQ4.117) and Ab8 (MQ4.112) were selected for further investigation.
The two selected antibodies were tested for binding to bovine collagen using ELISA. Both antibodies showed binding (Figure 2)

### Example 2

The hybridoma clones which produce MQ4.112 and MQ4.117 were investigated to deduce the antibody sequence (Table 2). First cells were lysed using Trizol. Next, RNA was isolated and antibody-coding sequences were amplified using the 5'-RACE approach. The product was then cloned into TA-cloning kit and the plasmid sequenced to identify the antibody sequence. The found VH and VL sequences, of mouse origin, were ordered in an expression vector with an hIgG1 backbone resulting in chimeric antibodies. Furthermore, the VH and VL domains were humanized using a dedicated program. Based on experience, one humanized sequence was chosen for each VH and VL domain and cloned into a hIgG1 expression vector. Additionally, for each domain, 1-3 sequences harboring point mutations were developed to check for antibody expression and function. All combinations of VH and VL were expressed (Table 3).

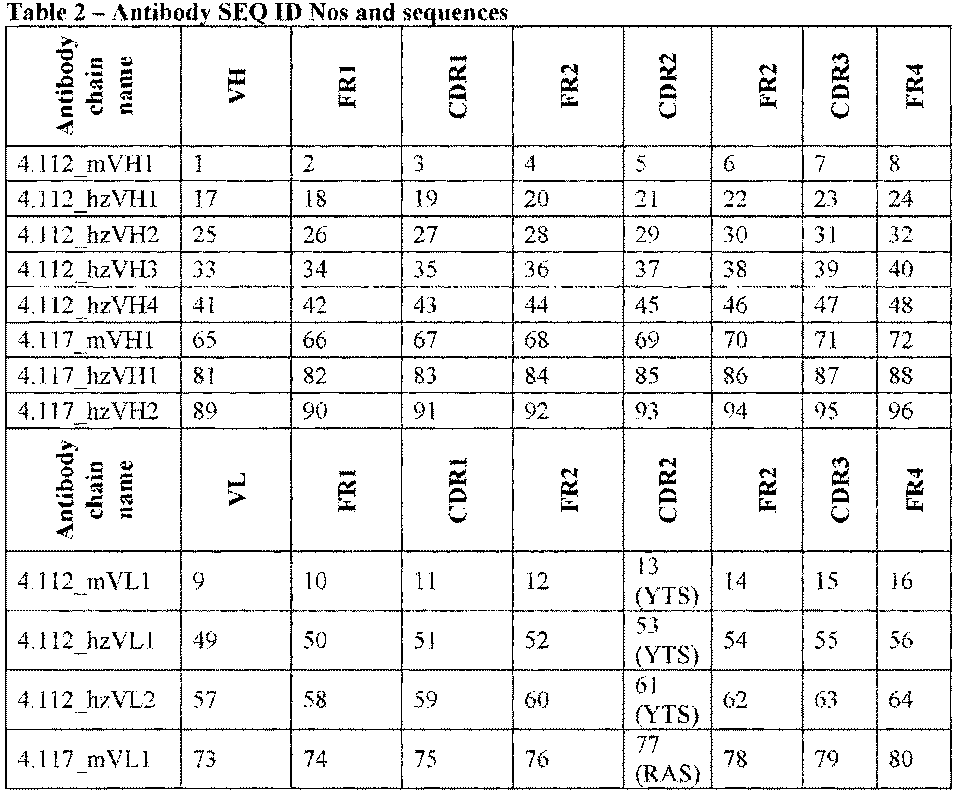

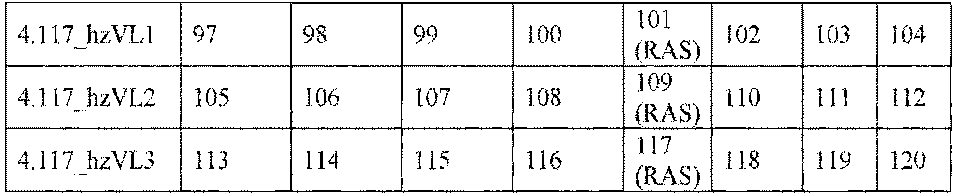

**Table 3 Antibodies**

| **Antibody name** | **VH** | **VL** | **Source** | **Isotype** |
|---|---|---|---|---|
| MQ4.112 | 4.112_mVH1 | 4.112_mVL1 | Hybridoma | Mouse |
| MQ4.112m_1_1 | 4.112_mVH1 | 4.112_mVL1 | Transient transfection | Mouse-human chimera |
| MQ4.112hz_1_1 | 4.112_hzVH1 | 4.112_hzVL1 | Transient transfection | Humanized |
| MQ4.112hz_1_2 | 4.112_hzVH1 | 4.112_hzVL2 | Transient transfection | Humanized |
| MQ4.112hz_2_1 | 4.112_hzVH2 | 4.112_hzVL1 | Transient transfection | Humanized |
| MQ4.112hz_2_2 | 4.112_hzVH2 | 4.112_hzVL2 | Transient transfection | Humanized |
| MQ4.112hz_3_1 | 4.112_hzVH3 | 4.112_hzVL1 | Transient transfection | Humanized |
| MQ4.112hz_3_2 | 4.112_hzVH3 | 4.112_hzVL2 | Transient transfection | Humanized |
| MQ4.112hz_4_1 | 4.112_hzVH4 | 4.112_hzVL1 | Transient transfection | Humanized |
| MQ4.112hz_4_2 | 4.112_hzVH4 | 4.112_hzVL2 | Transient transfection | Humanized |
| MQ4.117 | 4.117_mVH1 | 4.117_mVL1 | Hybridoma | Mouse |
| MQ4.117m_1_1 | 4.117_mVH1 | 4.117_mVL1 | Transient transfection | Mouse-human chimera |
| MQ4.117hz_1_1 | 4.117_hzVH1 | 4.117_hzVL1 | Transient transfection | Humanized |
| MQ4.117hz_1_2 | 4.117_hzVH1 | 4.117_hzVL2 | Transient transfection | Humanized |
| MQ4.117hz_1_3 | 4.117_hzVH1 | 4.117 hzVL3 | Transient transfection | Humanized |
| MQ4.117hz_2_1 | 4.117_hzVH2 | 4.117_hzVL1 | Transient transfection | Humanized |
| MQ4.117hz_2_2 | 4.117_hzVH2 | 4.117_hzVL2 | Transient transfection | Humanized |
| MQ4.117hz_2_3 | 4.117_hzVH2 | 4.117 hzVL3 | Transient transfection | Humanized |

### Example 3

The chimeric and humanized antibodies were tested for binding to bovine and human collagen type II. The mouse, chimera and human antibodies all show binding to Bovine Collagen type II (Figure 3). All humanized MQ4.112 seemed to react against collagen type II and higher reactivities are observed compared to mouse antibody. All humanized MQ4.117 antibodies seemed to react against collagen type II. Higher reactivities towards collagen type II was observed for mouse antibody compared to the humanized antibodies and the chimera. Inportantly, the mouse antibody was incubated with a mouse secondary HRP labeled antibody instead of the human secondary HRP labeled antibody which was used for all the other antibodies, this could cause the difference in absorbance.

### Methods for Examples 1 to 3

### Hybridoma development

Antibodies against bovine collagen type II have been raised in Balb/c mice. In short: At day 52 of the immunization procedure with bovine collagen II, serum samples were analyzed for bovine collagen type II reactivity. Spleens of responder mice were dissected following a final booster. Splenocytes were harvested and fused with a mouse myeloma cell-line (NS-1) using electrofusion. Supernatants of the resulting hybridoma cell lines were analyzed for collagen type II reactivity. A panel of primary clones have been selected and subcloned either by the use of limited dilution techniques or by ClonePix FL (Molecular Devices) automated subcloning device in order to ensure clonality.

### ELISA

Bovine collagen type II or Human collagen Type II was coated at 10-20 µg/mL in PBS in a 96-well high bind ELISA plate (Greiner bio-one, cat.#655061) overnight at 4 °C. Nonspecific binding was subsequently blocked using a 1 w/v% BSA (Sigma-Aldrich, cat.#A3912) in PBS solution (1%BSA) for 2 hours. The plate was washed 3 times with PBST before addition of 50 µL (of a serial dilution) of the antibodies. After 1 hour of incubation at room temperature, the plate was washed 5x with and subsequently incubated with 100 µL anti-mouse IgG conjugated with a HRP (ITK Diagnostics B.V) 1:5000 in PBS for 1 hour. After washing 3x PBST and 3x PBS, the plate was developed using 50 µl/well TMB substrate according to the manufacturer's recommendation and the absorbance was recorded after stopping the reaction by adding 50 µl/well 2 M H2SO4 at 450 nm on a Tecan Infinite F50 plate reader.

When chimera or humanized antibodies were tested, an anti-human IgG conjugated with a HRP (ITK Diagnostics B.V., cat.#2010-05) was used for detection.

### Humanization

Humanization of the mouse variable domains was performed using dedicated software (AbGenesis - AbProfile). Based on experience, one germline was selected for expression. Several sequences for every variable domain were ordered harboring point mutations to check for antibody expression and function.

### HEK293F cell culture

HEK293F cells (Invitrogen) were cultured in suspension in FreestyleTM 293 expression medium (Thermo Fisher Scientific, cat.#12338) at 37 °C, 110 rpm, 8 % CO2 and 95 % humidity. Cells were sub-cultured 3x-week at 0.3-0.5 million/mL in culture flasks. For counting and viability check an automated cell counter (Nexcelom Cellometer auto T4 Plus) was used and cells were diluted 1:1 in 0.4% Trypan Blue (Thermo Fisher Scientific, cat.#15250061).

### Transient antibody expression

Plasmids encoding for the antibodies were amplified in chemically competent *E. coli* XL1-blue bacteria and harvested using Mini/Midi/Maxi prep (Qiagen, cat.#27106, cat.#27191, cat.#12143; Sigma-Aldrich, cat.#NA0410). HEK293F cells (1 million/mL) were transfected with FectoPro transfection reagent (PolyPlus-transfection, cat.#116) 333 µg DNA/million cells of light chain plasmid and 167 µg/million cells of heavy chain plasmid for full size antibody expression. After 2.5 hours, transfection was boosted by addition of 2 mM Sodium butyrate. Cells were incubated at 37 °C, 110 rpm, 8 % CO₂ and 95 % humidity. After 4-6 days the cells were checked for viability and the antibody was harvested when the viability dropped below 60%, as detected using automated cell counting. To harvest the antibody, the culture was first centrifuged at 300 xg for 10 minutes at 4 °C to remove the cells and next at 4816 xg for 1 hour at 4 °C to remove cell debris and aggregates. The supernatant, containing the antibody in culture medium, was stored at 4 °C until further use. Antibody concentration was assessed using Octet measurements (ForteBio Octet Red96) with a Protein A coated sensor (Molecular Devices, cat.#18-5010) and a hIgG1 standard curve (Sartorius, cat.#18-1118). Typical concentrations ranged between 0.01 and 0.4 mg/mL.

### Antibody purification

Antibodies were purified using mAbs Select Sure Resin (Cytiva) according to the manufacturer's instructions using centrifugation without brake. In short, resin was equilibrated with 4x 5 bed volumes of Binding buffer (20mM phosphate buffer, 150mM NaCl, pH7.59). Based on the expected antibody yield, as measured using Octet, add 1 mL beads solution (1:1 beads:buffer) per 15 mg of antibody. Incubate overnight at 4 °C on a tube roller. The next day, centrifuge sample at 300 xg for 15 minutes and take off the supernatant. Resuspend beads in binding buffer and load onto a pre-wetted polypropylene column. Wash the resin with 4x 5 CV of binding buffer. Elute the antibody using 5x 1 CV of elution buffer (100mM sodium citrate-HCl pH 3.0) and add neutralization buffer (1M Tris-HCl pH8.46) to reach a neutral pH. Antibody concentration is determined using Nanodrop. If needed, antibodies are dialysed against PBS using a Slide-A-LyzerTM G2 Dialysis Cassette according to the manufacturer's protocol. Next, antibodies are filtered (0.22 µm) and concentration is determined using Nanodrop measurement.

### Radiolabeling of antibodies with ¹²⁵I and ¹¹¹In

¹²⁵I labeling: Collagen type II antibodies and an isotype control IgG2b antibody were radiolabeled using the chloramine-T method. Hypochlorite released from chloramines T acts as an effective oxidizing agent for iodine to form iodine monochloride which undergoes electrophilic substitution with aromatic rings in amino acid tyrosine. 125I protein was separated from free ¹²⁵I by sephadex G-25 fractionation. The specific activity was 3 µCi/µg. ¹¹¹In labeling: collagen type II and isotype control antibodies were dissolved in 0.25 M ammonium acetate, pH 5.4. For radiolabeling, 70 µg of antibodies was incubated with 260 Mbq of ¹¹¹In-oxine (Covidien BV) in 0.1 M 2-(N-morpholino) ethanesulfonic acid buffer ,pH 5.4 (twice the volume of ¹¹¹In), for 15 min at 90o C. After incubation 50 mM EDTA was added to a final concentration of 5 mM. Subsequently, the reaction mixture was purified on a PD10 column, eluted with PBS containing 0.5% BSA. ¹¹¹In collagen type II and isotype control antibody was diluted in PBS containing 0.5% lysozyme. Labeling efficiency and radiochemical purity were determined with ITLC and HPLC.

### In vitro cartilage binding studies

Bovine and human cartilage explants were cut and placed into PBS in 24-wells plates, each well contained 3 randomly picked explants. Bovine cartilage explants were treated with various concentrations of trypsin (0%, 0.6%, 1.25% and 2,5%) in PBS for 1 hour at 37°C. Thereafter, trypsin was inactivated by incubating the explants with 1% fetal calf serum in PBS for 15 minutes and were then thoroughly washed. Explants were incubated for 3 hours with 5µg/ml of ¹²⁵I or ¹¹¹In labelled antibodies at 37°C or with 5µg/ml HRP labelled antibodies overnight at 4°C. Thereafter, explants were washed in PBS and standard-sized pieces were obtained using a 3 mm punch (Stiefel Laboratories, Coral Gables, Florida, USA). This also eliminated the unintended binding of antibodies to exposed collagen as a result of the dissection of the explants. Explants were then counted for activity in a gammacounter and analyzed using RiaCalc WIZ program 3.6. Explants incubated with HRP labelled antibodies were incubated with DAB for 10 minutes and then washed with water.

Thereafter photographs were taken and the mean staining intensity corrected for total area was calculated using ImageJ software.

### Example 4 - Clone selection on bovine cartilage - Binding of the anti-COLL II antibodies to bovine cartilage with or without trypsin-induced damage.

### Methods

### Cartilage processing

Bovine cartilage from the metacarpophalangeal joint was isolated from a fresh cow leg (COW 5017) obtained from slaughterhouse waste. The bovine cartilage was isolated from the joint with a surgical blade and 4 mm punches were taken with a biopsy punch. Cartilage explants were kept overnight (o/n) in Dulbecco's Modified Eagle's Medium/Nutrient Mixture F12 Ham (DMEM/F12) media without fetal bovine serum (FBS) supplemented with 10.000 U/mL penicillin/streptomycin at 37°C with 5% CO₂. The next day, cartilage punches were washed with 1x phosphate buffered saline (PBS) (twice for 5 minutes (min)). Half of the explants were incubated for 1 hour (H) with 2.5% trypsin-EDTA (GIBCO 15400-054) in PBS at 37°C on a roller in a 50 ml falcon tube. The other half was incubated under the same conditions in PBS. The trypsin-EDTA was replaced by 1% FBS in PBS, in which the explants were incubated for 15 min. Cartilage explants were washed with PBS (3x 5 min).

### Staining and explant storage/histology

For each condition, 3 cartilage explants were distributed over 50 ml falcon tubes. The explants were incubated with 5 µg/ml primary antibodies in PBS supplemented with 1% normal rabbit serum in PBS (see table 1) for 1 H at room temperature (RT) on a shaker. Cartilage explants were washed with PBS (3x 30 min). The cartilage explants were incubated with 1:200 diluted HRP-conjugated secondary antibody in 1% normal rabbit serum in PBS for 1 H at RT on a shaker (Table 1). Cartilage explants were washed with PBS (2x 15 min) followed by an o/n wash with PBS at 4°C. The cartilage explants were incubated with 3,3'-diaminobenzidine (DAP) for 10 min at RT. Cartilage explants were washed twice with tap water. Explants were dried on a tissue and pictures were taken. The cartilage was fixed in formalin (4 H) and transferred to 70% ethanol until processed/embedded in paraffin. Sections (5 mm) were cut, mounted and stained with safranin O (SafO) (2x 5 min Xylene, 5 min ethanol 100%, 5 min ethanol 96%, 5 min ethanol 70%, rinse with ultra-pure water, 15 min SafO (BDH 343122N) 0.1% in ultra-pure water, rinse with ultra-pure water, 5 min Fast Green FCF 0.1% (BDH 340304F) in ultra-pure water, 2x short rinse in ethanol 96%, 5 min ethanol 100%, 2x 5 min Xylene) and sealed with Permount (Fisher Scientific SP15-500). Pictures were taken with a Leitz DMRD microscope (Leica Microsystems).

**Table 1: staining conditions**

| **Tube i.d.** | **Primary antibody** | **Secondary antibody** |
|---|---|---|
| 1NT | MQ4.112 | HRP-conjugated II antibody rabbit anti mouse |
| 1T | | |
| 2NT | MQ4.117 | HRP-conjugated II antibody rabbit anti mouse |
| 2T | | |
| 3NT | MQ4.112(m_1_1) | Human backbone: rabbit anti human IgG HRP |
| 3T | | |
| 4NT | MQ4.117 (m_1_1) | Human backbone: rabbit anti human IgG HRP |
| 4T | | |
| 5NT | MQ4.112hz_3_1 | Human backbone: rabbit anti human IgG HRP |
| 5T | | |
| 6NT | MQ4.117hz_1_2 | Human backbone: rabbit anti human IgG HRP |
| 6T | | |
| 7NT | MQ20.101 (murine isotype) | HRP-conjugated II antibody rabbit anti mouse |
| 7T | | |
| 8NT | 2.201a (human isotype) | Human backbone: rabbit anti human IgG HRP |
| 8T | | |
| 9NT | X | HRP-conjugated II antibody rabbit anti mouse |
| 9T | | |
| 10NT | X | Human backbone: rabbit anti human IgG HRP |
| 10T | | |
| 11NT | X | X |
| 11T | | |

| | | |
|---|---|---|
| NT= no trypsin, T=trypsin treated, m= murine, vc=variable chain and hz= fully humanized. | | |

### Results

Binding of the original murine (MQ4.112 and MQ4.117), variable domain of original mouse murine clones in a human backbone (MQ4.112(m_1_1) and MQ4.117(m_1_1)) and the fully humanized clones (MQ4.112hz_3_1 and MQ4.117hz_1_2) was tested on bovine cartilage punches which were either treated with trypsin to induce cartilage damage/proteoglycan depletion or left untreated. Murine clone MQ4.112 show more binding to trypsin-treated bovine cartilage compared to non-trypsin treated cartilage. Similar results are observed for the partially and fully humanized MQ4.112 antibodies. No binding of isotype control antibodies (MQ20.101 (murine isotype control) and 2.201a (human isotope control) or only secondary antibodies was observed (Figure 6A). SafO staining on sections of these cartilage explants reveals trypsin-induced proteoglycan depletion (PG) (Figure 6B).

### Conclusion

The collagen type II antibody clone MQ4.112 can be used to detect trypsin-induced cartilage damage in bovine cartilage. After fully humanizing this clone it still can be used to detect trypsin-induced cartilage damage in bovine cartilage. The isotype controls or only secondary antibodies did not bind.

### Example 5 - Clone selection with [¹¹¹In]In-DTPA-coniugated anti-COLL II antibodies - Binding of ¹¹¹In-labeled DTPA-conjugated anti-COLL II antibodies to bovine and human cartilage (with or without trypsin-induced damage).

### Methods

### Bovine cartilage explants and gamma counting

Bovine cartilage from the metacarpophalangeal joint was isolated from a fresh cow leg obtained from slaughterhouse waste. Cartilage punches (4 mm) were made using a biopsy punch, divided over 50 ml falcon tubes (3 explants/tube) and incubated o/n in DMEM/F12 without fetal bovine serum (FBS) supplemented with 10.000 U/mL penicillin/streptomycin (final volume 1 ml) at 37°C with 5% CO₂. The next day, cartilage punches were washed once with PBS. The explants were incubated for 1 H with 2.5% Trypsin-EDTA in PBS or PBS (final volume 1 ml) at 37°C on a roller. The solution was replaced by 1% FBS in PBS, in which the explants were incubated for 15 min. Cartilage explants were washed with PBS on a shaker (2x 5 min). Three explants/condition were exposed 4 µg/ml [¹¹¹In]In-DTPA-IrDye 800CW anti-COLL II antibody (aimed for 0.5 MBq/µg antibody) (Table 2) in DMEM /F12 supplemented with 0.5% BSA (final volume was 1 ml) for 4 H on a shaker at 37°C. The antibodies were labeled with [¹¹¹In]InCl₃ (Covidien BV, Petten, the Netherlands) (0.5 MBq/µg antibody) and purified with a PD10 spin column to remove free ¹¹¹In (Table 2). Cartilage explants were washed with PBS (50 ml) on a shaker at 37°C (2x 15 min), followed by an o/n wash with PBS (50 ml) at RT on a shaker. The bovine cartilage explants were transferred to biodistribution tubes which were individually weight empty and when containing a cartilage punch. The amount of ¹¹¹In associated to the cartilage was measured using an automated, well-type γ-counter (WIZARD², 2480 Automatic Gamma Counter, Perkin Elmer, US).

**Table 2: Antibody exposure conditions**

| **Tube i.d.** | **DTPA-conjugated antibody** | **MBq/µg antibody after PD10 spin column purification** |
|---|---|---|
| 1NT | MQ4.112 | 0,35 |
| 1T | | |
| 2NT | MQ4.117 | 0,44 |
| 2T | | |
| 3NT | MQ4.112(m_1_1) | 0,35 |
| 3T | | |
| 4NT | MQ4.117 (m_1_1) | 0,43 |
| 4T | | |
| 5NT | MQ4.112hz_3_1 | 0,47 |
| 5T | | |
| 6NT | MQ4.117hz_1_2 | 0,50 |
| 6T | | |
| 7NT | MQ20.101 (murine isotype) | 0,45 |
| 7T | | |
| 8NT | 2.201a (human isotype) | 0,53 |
| 8T | | |

| | | |
|---|---|---|
| NT= no trypsin, T=trypsin treated, m= murine, vc=variable chain and hz= fully humanized. | | |

### Human cartilage explants and gamma counting

From surgical waste material obtained after total knee arthroplasty (Patient number SMK1195), human cartilage from the macroscopically preserved regions was isolated with a surgical blade, and 6 mm punches were made using a biopsy punch. Human cartilage explants were overnight incubated in DMEM/F12 without FBS supplemented with 10.000 U/mL penicillin/streptomycin and 1 mM sodium pyruvate (Invitrogen 11360088) at 37°C with 5% CO₂. Half of the explants were incubated for 1 H with 2.5% Trypsin-EDTA in PBS at 37°C on a shaker in a 50 ml falcon tube (final volume 2 ml). The other half was incubated under the same conditions in PBS. The trypsin-EDTA was replaced by 1% FBS in PBS (to block the enzymatic activity of trypsin), in which the explants were incubated for 15 min. Cartilage explants were washed with PBS on a shaker (2x 5 min). Three explants/ condition were exposed to 4 µg/ml DTPA-isotype (2.201a) or DTPA-anti-COLL II antibody (MQ4.112 hz3_1) in DMEM/F12 media supplemented with 0.5% BSA (final volume was 1 ml) for 1.5 H on a shaker at 37°C. The antibodies were labeled with [¹¹¹In]InCl₃ (Covidien BV, Petten, the Netherlands) (aimed for 0.2 MBq/µg antibody) and purified with a PD10 spin column to remove free ¹¹¹In (Table 3). After this incubation the samples were washed with PBS (50 ml) at 37°C on a shaker (2x 15 min), followed by an o/n wash with PBS on a shaker at RT. The human cartilage explants were transferred to biodistribution tubes (containing 1 ml of PBS) which were individually weight without and with a cartilage punch. The amount of ¹¹¹In associated to the cartilage was measured using an automated, well-type γ-counter (WIZARD², 2480 Automatic Gamma Counter, Perkin Elmer, US). The cartilage was fixed in formalin (4 H) and transferred to 70% ethanol until processed/embedded in paraffin.

**Table 3: Antibody exposure conditions**

| **Tube i.d.** | **DTPA-conjugated antibody** | **MBq/µg antibody after PD10 spin column purification** |
|---|---|---|
| 1NT | MQ4.112hz_3_1 | 0,233 |
| 1T | | |
| 2NT | 2.201a (human isotype) | 0,257 |

| | | |
|---|---|---|
| NT= no trypsin, Trtrypsin treated and hz= fully humanized. | | |

### Results

In general, there is more binding of the anti-COLL II antibodies to trypsin-treated cartilage compared to untreated bovine cartilage. MQ4.117 shows more binding to undamaged cartilage compared to MQ4.112. For the fully humanized clone, MQ4.112hz_3_1 binds more efficiently to damaged bovine cartilage compared to MQ4.117hz_2. The original murine antibodies (both clones) bind more to (damaged)-bovine cartilage compared to their fully humanized antibodies. We clearly can discriminate trypsin-damaged bovine cartilage with the fully humanized MQ4.112hz_3_1 antibody (third pair of bars in Figure 7).

The cartilage associated radioactivity corrected for their weight of [¹¹¹In]In-DTPA-MQ4.112hz_3_1 is higher in trypsin-damaged human cartilage compared to non-treated explants. Moreover, the radioactivity in human cartilage explants of the isotype antibody was very low independent of trypsin-treatment (Figure 8).

### Conclusion

MQ4.112hz_3_1 is selected for future experiments as it discriminates between trypsin-damaged and undamaged cartilage. This COLL II antibody (MQ4.112hz_3_1) binds more to trypsin-treated cartilage compared to untreated cartilage, both on bovine and human explants. This indicates that it does detect trypsin induced cartilage damage. The isotype control antibodies do not bind.

### Example 6 - Binding of COLL II antibody to human damaged cartilage (trypsin) - Binding of the anti-Coll II antibodies (DTPA-IrDye 800CW-coniugated) to human cartilage with or without trypsin-induced damage.

### Methods

### Human cartilage explants and binding assay

From surgical waste material obtained after total knee arthroplasty (Patient number SMK1186), human cartilage from the macroscopically preserved regions was isolated with a surgical blade, and 6 mm punches were made using a biopsy punch. Human cartilage explants were overnight incubated in DMEM/F12 without FBS supplemented with 10.000 U/mL penicillin/streptomycin at 37°C with 5% CO₂. Half of the explants were incubated for 1 H with 2.5% Trypsin-EDTA in PBS at 37°C on a roller in a 50 ml falcon tube. The other half was incubated under the same conditions in PBS. The trypsin-EDTA was replaced by 1% FBS in PBS (to block the enzymatic activity of trypsin), in which the explants were incubated for 15 min. Cartilage explants were washed with PBS (3x 5 min). Three explants/condition were divided over sterile FACS tubes and exposed 4 µg/ml DTPA-IrDye 800CW isotype (2.201a) (substitution ratio IrDye 800CW= 1.54) or DTPA-IrDye 800CW anti-COLL II antibody (MQ4.112 hz3_1) (substitution ratio IrDye 800CW= 1.83) in DMEM/F12 media supplemented with 0.5% BSA (final volume was 1 ml) for 4 H on a roller at 37°C. After this incubation the samples were transferred to 50 ml falcon tubes (1 tube/condition, 3 punches/tube) and washed with PBS (50 ml) at 37°C (1x 30 min), followed by an o/n wash with PBS on a shaker at 4°C.

### Acquisition with Odyssey CLX imager, histology and storage

The explants were transferred to the bottom of wells of a 96-well plate to image with the Odyssey CLX near infrared imaging system. The fluorescent signal was measured in manually drawn ROI around the cartilage explants which was corrected for background signal by the software. The background corrected intensity of the cartilage explants was divided by its area, the intensity/pixel was plotted. The cartilage was fixed in formalin (4 H) and transferred to 70% ethanol until processed/embedded in paraffin. Sections (5 mm) were cut, mounted and stained with SafO (2x 5 min Xylene, 5 min ethanol 100%, 5 min ethanol 96%, 5 min ethanol 70%, rinse with ultra-pure water, 15 min SafO (BDH 343122N) 0.1% in ultra-pure water, rinse with ultra-pure water, 5 min Fast Green FCF 0.1% (BDH 340304F) in ultra-pure water, 2x short rinse in ethanol 96%, 5 min ethanol 100%, 2x 5 min Xylene) and sealed with Permount (Fisher Scientific SP15-500). Pictures were taken with a Leitz DMRD microscope.

### Results

Minor proteoglycan depletion (PG) depletion in the preserved region of OA cartilage of this patient could be the reason for binding of the DTPA-IrDye 800CW anti-COLL II (MQ4.112 hz3_1) antibody to these explants. The anti-COLL II antibody shows increased binding to trypsin-induced damaged human cartilage explants compared to untreated explants obtained from the preserved regions of OA cartilage. The isotype antibody gave no to very low signal in both trypsin-treated and untreated human cartilage explants. SafO staining on sections of these cartilage explants reveals trypsin-induced PG (Figure 9C).

### Conclusion

After dually conjugating the anti-COLL II antibody (MQ4.112hz_3_1) with DTPA and IrDye 800CW it still can be used to detect trypsin-induced cartilage damage/proteoglycan depletion on human cartilage of the preserved region of an OA patient.

Example 7 - **Binding of [¹¹¹In]In-DTPA-IrDye 800CW-anti COLL II to damaged cartilage (trypsin) *in vivo*** - Binding of the selected anti-COLL II antibody ([¹¹¹In]In-DTPA-IrDye 800CW-anti COLL II, clone MQ4.112 hz3_1) to human cartilage with or without trypsin-induced damage implanted in SCID mice *in vivo.*

### Methods

### Human cartilage explants collection and trypsin treatment

From surgical waste material obtained after total joint arthroplasty (table 4), human cartilage from the macroscopically preserved regions was isolated with a surgical blade, and 6 mm punches were made using a biopsy punch. Human cartilage explants were cultured in DMEM/F12 with 10% FBS supplemented with 10.000 U/mL penicillin/streptomycin at 37°C with 5% CO₂ until used (final volume 5 mL in 6-well plates). Cartilage punches were washed with PBS (50 ml) for 4 H at 37°C on a roller. Per cartilage donor, half of the explants were incubated for 1 H with 2.5% Trypsin-EDTA in PBS at 37°C on a roller in a 50 ml falcon tube (final volume 10 ml). The other half was incubated under the same conditions in PBS. In each tube, the trypsin/PBS-solution was replaced by 1% FBS in PBS (to block the enzymatic activity of trypsin), in which the explants were incubated for 15 min on a roller at 37°C. Cartilage explants were washed with PBS (50 ml) (2x 2 H) and kept o/n in PBS.

**Table 4: collection of human cartilage for in vivo imaging.**

| Patient i.d. | Diagnosis | Punches obtained | Hospital | Joint | Amount of days in culture before implant |
|---|---|---|---|---|---|
| 383 | OA due to polyarthritis | 11 | RUMC | Knee | 2 |
| 1220 | OA | 10 | SMK | Knee | 2 |
| 1221 | OA | 10 | SMK | Knee | 2 |
| 983 | OA | 9 | RUMC | Hip | 1 |

### Cartilage explant implantation, biodistribution and IVIS imaging

Twelve female CB17-SCID mice (CB17/lcr-Prkdcscid/Rj) (Janvier Laboratories, France) were group-housed (6/cage, 3/cage after cartilage implantation) in individually-ventilated cages on a 12 H day-night cycle with *ad libitum* access to standard chow and water. All animal experiments were conducted according to Dutch law and approved by the Dutch Central Authority for Scientific Procedures on Animals (AVD 103002016786 and AVD 10300202010389). At day 0 of the experiment, mice were anaesthetized with 2.5% isoflurane with a constant flow of oxygen as carrier. Mice were shaved in the neck region and a small central incision was made. Using a scissor, 2 subcutaneous pockets were created in the shoulder region. From the same cartilage donor, one preserved cartilage explant was implanted subcutaneously on the left and one trypsin-damaged explant on the right side of the mice. The wounds were closed with a surgical clips. Mice number 4, 5 and 6 received an intra-articular zymosan injection (6 µl 180 µg) in the right knee and a saline (6 µl) injection in the left knee. At day 6, the DTPA-IrDye 800CW (substitution ratio IrDye 800CW= 1.83) anti-COLL II antibody (MQ4.112 hz3_1) was used for ¹¹¹In labeling. At day 7, the mice per cartilage donor were randomly divided over the 3 dosages and were intra-venously injected with the allocated dose (200 µl). At day 10 (after 72 H), the mice were sacrificed with CO₂ and the skin on the back of the mice was opened so that the cartilage explants became visible. The mice were scanned in the IVIS imaging system (exposure: 1 min, Ex: 745nm, Em: ICG, + 1 min Ex: 640 nm, Em: ICG for background). Blood (by heart puncture), muscle, cartilage explant on the left, cartilage explant on the right, heart, lung, spleen, pancreas, kidney, liver, stomach, duodenum, patella, top of tibia bone (side of the knee joint) and femur edge (side in the knee joint) were collected for biodistribution of the antibody. The collected tissues were weight and the radioactivity was measured using an automated, well-type γ-counter (WIZARD², 2480 Automatic Gamma Counter, Perkin Elmer, US).

### Acquisition with Odyssey CLX imager, histology and storage

The cartilage explants were fixed in formalin (4 H) and transferred to 70% ethanol until processed/embedded in paraffin. Each explant is divided in 2 and both halves are embedded in the same paraffin block with the middle of the explant face down. Sections (5 mm) were cut and in order to include sections at different depths 1 section every 20 sections is mounted. Fluorescence in these sections was determined with the Odyssey near infrared imaging system (scan intensity 3 (for both the 700 and 800 channel) with a focus distance of 1 mm with a resolution of 21 µm. Thereafter the same sections were stained with SafO (2x 5 min Xylene, 5 min ethanol 100%, 5 min ethanol 96%, 5 min ethanol 70%, rinse with ultra-pure water, 15 min SafO (BDH 343122N) 0.1% in ultra-pure water, rinse with ultra-pure water, 5 min Fast Green FCF 0.1% (BDH 340304F) in ultra-pure water, 2x short rinse in ethanol 96%, 5 min ethanol 100%, 2x 5 min Xylene) and sealed with Permount (Fisher Scientific SP15-500). Pictures were taken with a Leitz DMRD microscope.

**Table 5: experimental details in vivo**

| Cage number | Mice number | Cartilage donor | Antibody dose (µg) |
|---|---|---|---|
| 1 | 1 | #383 | 3 |
| | 2 | | 30 |
| | 3 | | 10 |
| 2 | 4 | #1220 + intra-articular zymosan injection | 3 |
| | 5 | | 10 |
| | 6 | | 30 |
| 3 | 7 | #1221 | 30 |
| | 8 | | 3 |
| | 9 | | 10 |
| 4 | 10 | #983 | 3 |
| | 11 | | 30 |
| | 12 | | 10 |

### Results

There is uptake of the [¹¹¹In]In-DTPA-IrDye 800CW anti-COLL II (MQ4.112hz3_1) in the human cartilage implanted in the SCID mice. The fluorescent signal could be detected and was more in trypsin-treated cartilage explants compared to untreated cartilage (Figure 10A). In addition, the %injected dose corrected for the gram of the cartilage explants was significantly higher in trypsin treated explants compared to untreated explants (Figure 10B), although in the group with 10 µg injected antibody this did not reach statistical significance. In sections of the untreated cartilage explants we were able to detect fluorescent signal from the anti-COLL II antibody on the surface of the explant, this might represent minor damage to the macroscopically defined preserved cartilage from OA joints which was used to implant (Figure 10C). The fluorescent signal was higher in trypsin treated explants (Figure 10C).

### Conclusion

There is uptake of the [¹¹¹In]In-DTPA-IrDye 800CW anti-COLL II (MQ4.112hz3_1) in the human cartilage implanted in the SCID mice. Furthermore, binding is higher in the cartilage damaged with trypsin compared to untreated cartilage explants. No strong differences are observed between doses of antibody.

Example 8 - Binding of DTPA-IrDye 800CW-anti COLL II to different OA-damage stages *ex vivo* - Binding of the selected anti-COLL II antibody (DTPA-IrDye 800CW-anti COLL II, clone MQ4.112 hz3_1) to human cartilage with different stage of OA-induced damage *ex vivo.*

### Methods

### Human cartilage explants and binding assay

From surgical waste material obtained after total knee arthroplasty (Patient number SMK1180), human cartilage from the macroscopically preserved, medium damage and severe damaged regions were isolated with a surgical blade. Human cartilage explants were overnight incubated in DMEM/F12 without FBS supplemented with 10.000 U/mL penicillin/streptomycin at 37°C with 5% CO₂. Punches (6 mm) were generated using a biopsy punch. Three explants/damage condition were divided over sterile FACS tubes and exposed 4 µg/ml DTPA-IrDye 800CW isotype (2.201a) (substitution ratio IrDye 800CW= 1.54) or DTPA-IrDye 800CW anti-COLL II antibody (MQ4.112 hz3_1) (substitution ratio IrDye 800CW= 1.83) in DMEM/F12 media supplemented with 0.5% BSA (final volume was 1 ml) for 4 H on a roller at 37°C. After this incubation the samples were transferred to 50 ml falcon tubes (1 tube/condition, 3 punches/tube) and washed with PBS (50 ml) at 37°C (1x 30 min), followed by an o/n wash with PBS on a shaker at 4°C.

### Acquisition with Odyssey CLX imager, histology and storage

The explants were transferred to the bottom of wells of a 96-well plate to measure the cartilage associated fluorescence with the Odyssey CLX near infrared imaging system (Figure 11). The cartilage was fixed in formalin for 4 H and thereafter transferred to 70% ethanol until processed/embedded in paraffin. Sections (5 mm) were cut, mounted and stained with SafO (2x 5 min Xylene, 5 min ethanol 100%, 5 min ethanol 96%, 5 min ethanol 70%, rinse with ultra-pure water, 15 min SafO (BDH 343122N) 0.1% in ultra-pure water, rinse with ultra-pure water, 5 min Fast Green FCF 0.1% (BDH 340304F) in ultra-pure water, 2x short rinse in ethanol 96%, 5 min ethanol 100%, 2x 5 min Xylene) and sealed with Permount (Fisher Scientific SP15-500). Pictures were taken with a Leitz DMRD microscope.

### Results

The DTPA-IrDye 800CW anti-COLL II antibody binds more to cartilage when OA-damage increases, the isotype control did not bind regardless of the OA-damage severity (Figure 11 top). We then determined the localization of the fluorescent signal in sections of these explants, and reveal that the fluorescent signal localizes on the spots where visible cartilage damage is present (Figure 11 bottom). Higher fluorescent signal localizes where SafO staining revealed PG depletion (loss of red staining).

### Conclusion

DTPA-IrDye 800CW anti-COLL II antibody is able to distinguish different levels of disease induced damage to cartilage within and between patient samples.

## Claims

1. An antibody, or an antigen-binding fragment thereof, that binds to collagen type II, wherein the antibody comprises a set of three heavy chain complementarity determining regions (CDRH1, CDRH2 and CDRH3) and three light chain complementarity determining regions (CDRL1, CDRL2 and CDRL3), wherein the set of CDRH1, CDRH2, CDRH3 amino acid sequences is selected from:
(a) SEQ ID NOs: 35, 37 and 39;
(b) SEQ ID NOs: 3, 5 and 7;
(c) SEQ ID NOs: 19, 21 and 23;
(d) SEQ ID NOs: 27, 29 and 31;
(e) SEQ ID NOs: 43, 45 and 47;
(f) SEQ ID NOs: 67, 69 and 71;
(g) SEQ ID NOs: 83, 85 and 87; and
(h) SEQ ID NOs: 91, 93 and 95;
and the set of CDRL1, CDRL2, CDRL3 amino acid sequences is selected from:
(a) SEQ ID NOs: 51, 53 and 55;
(b) SEQ ID NOs: 11, 13 and 17;
(c) SEQ ID NOs: 59, 61 and 63;
(d) SEQ ID NOs: 75, 77 and 79;
(e) SEQ ID NOs: 99, 101 and 103;
(f) SEQ ID NOs: 107, 109 and 111; and
(g) SEQ ID NOs: 115, 117 and 119.

2. The antibody or fragment thereof of claim 1, wherein the antibody or fragment thereof is coupled to a detectable label.

3. The antibody or fragment thereof of claim 2 wherein the detectable label is a fluorescent moiety, a superparamagnetic iron oxide nanoparticle (SPION), or a radionucleotide.

4. The antibody or fragment thereof of any one of claims 1 to 3, that is a bispecific antibody comprising one antigen binding domain according to claim 1 or claim 2 that binds to collagen type II, and a second antigen binding domain that is or that binds to a growth factor, optionally NGF, FGF1-4, FGF-18, IGF-1, BMP-2, BMP-6, BMP-7, PDGF, TGFBeta3.

5. The antibody or fragment thereof of any one of claims 1 to 4, wherein
(a) the antibody is fused to a therapeutic agent, optionally via a cleavable linker, optionally a matrix-metalloproteinase (MMP) cleavable linker; or
(b) the antibody is conjugated to an immunoliposome, optionally wherein the immunoliposome contains a therapeutic agent;
optionally wherein the therapeutic agent of (a) or (b) is a therapeutic agent for treating osteoarthritis or rheumatoid arthritis, optionally wherein the therapeutic agent is a growth factor or an anti-inflammatory cytokine.

6. A pharmaceutical composition comprising the antibody or fragment thereof according to claim 4 to claim 5.

7. A method of treating a subject comprising administering to the subject a therapeutically effective amount of the antibody or fragment thereof according to claim 4 or claim 5, or the pharmaceutical composition of claim 6.

8. The method according to claim 7, wherein the method is for treating osteoarthritis or rheumatoid arthritis.

9. A method of detecting, quantifying or imaging cartilage damage or loss of cartilage proteoglycan, the method comprising:
(i) contacting the cartilage with an antibody according to any one of claims 1 to 5; and
(ii) detecting, quantifying or imaging the presence of the antibody bound to the cartilage,
wherein the presence of antibody bound to cartilage, or increased presence of antibody bound to the cartilage compared to a reference, indicates cartilage damage or loss of cartilage proteoglycan.

10. A method of diagnosing, prognosing or monitoring progression or regression of osteoarthritis or rheumatoid arthritis in a subject, the method comprising
(i) administering to the subject an antibody or fragment thereof coupled to an detectable label according to claim 2 or claim 3; and
(ii) detecting, quantifying or imaging the antibody or fragment thereof coupled to detectable label bound to cartilage in the subject,
wherein
(I) the presence of the antibody bound to cartilage, or of increased antibody bound to cartilage compared to a reference, indicates osteoarthritis or rheumatoid arthritis in the subject;
(II) increased antibody bound to cartilage in the subject compared to a reference or to earlier measurements from the same subject indicates progression of osteoarthritis or rheumatoid arthritis or a negative prognosis; or
(III) decreased antibody bound to cartilage in the subject compared to a reference or to earlier measurements from the same subject indicates regression of osteoarthritis or rheumatoid arthritis or a positive prognosis in the subject.

11. A method of monitoring the response of a subject to a treatment for osteoarthritis or rheumatoid arthritis, wherein the subject has been administered an antibody according to claim 2 or claim 3, the method comprising detecting, quantifying or imaging the antibody bound to the cartilage in the subject (a) before administration of the treatment and (b) after administration of the treatment and comparing (a) and (b).

12. The antibody of claim 2 or claim 3 for use in a method according to any one of claims 9 to 11.

13. One or more polynucleotides encoding the antibody or fragment thereof according to any one of claims 1 to 5.

14. One or more vectors comprising the polynucleotide or polynucleotides of claim 13 or a host cell comprising said vector or vectors.

15. A method for producing an antibody or fragment thereof according to according to any one of claims 1 to 5, the method comprising culturing the host cell of claim 14 and isolating the antibody or fragment thereof from the culture.
